# EUROPEAN PATENT SPECIFICATION

(11) **EP 1 791 976 B1**
(45) Date of publication and mention of the grant of the patent: **28.10.2009**
(21) Application number: 05783566.2
(22) Date of filing: 27.08.2005
(51) Int. Cl.: C12Q 1/68

(54) **ASSOCIATION OF PROTEIN POLYMORPHISMS WITH CORONARY HEART DISEASE**
ASSOZIATION VON PROTEINPOLYMORPHISMEN MIT KORONARER HERZKRANKHEIT
ASSOCIATION DE POLYMORPHISMES PROTEINIQUES ET D'UNE CORONAROPATHIE

(30) Priority: 10.09.2004 EP 04021528
(43) Date of publication of application: 06.06.2007
(73) Proprietor: Sanofi-Aventis Deutschland GmbH, 65929 Frankfurt am Main (DE)
(72) Inventor: KOZIAN, Detlef, 65779 Kelkeim (DE); GOEGELEIN, Heinz, 60528 Frankfurt (DE); SIEGLER, Karl-Ernst, 67069 Ludwigshafen (DE); JACOBS, Martina, 68165 Mannheim (DE); DELEUZE, Jean-François, F-77380 Combs La Ville (FR); RICARD, Sylvain, F-75013 Paris (FR); MACE, Sandrine, F-78350 Jouy-en-Josas (FR)
(86) International application number: PCT/EP2005/009270
(87) International publication number: WO 2006/027128

(56) References cited:
- EP-A- 0 764 721
- WO-A-97/18308
- WO-A-2004/018701
- DATABASE EMBL [Online] 29 November 1995 (1995-11-29), "Human gene for inward rectifier K channel, complete cds." XP002343983 retrieved from EBI Database accession no. D50582
- JERON A ET AL: "KCNJ11 polymorphisms and sudden cardiac death in patients with acute myocardial infarction." JOURNAL OF MOLECULAR AND CELLULAR CARDIOLOGY, vol. 36, no. 2, February 2004 (2004-02), pages 287-293, XP002344211 ISSN: 0022-2828
- BIENENGRAEBER MARTIN ET AL: "ABCC9 mutations identified in human dilated cardiomyopathy disrupt catalytic KATP channel gating" NATURE GENETICS, vol. 36, no. 4, April 2004 (2004-04), pages 382-387, XP002344234 ISSN: 1061-4036
- GLOYN ANNA L ET AL: "Activating mutations in the gene encoding the ATP-sensitive potassium-channel subunit Kir6.2 and permanent neonatal diabetes." THE NEW ENGLAND JOURNAL OF MEDICINE. 29 APR 2004, vol. 350, no. 18, 29 April 2004 (2004-04-29), pages 1838-1849, XP008052327 ISSN: 1533-4406
- REIMANN F ET AL: "STRUCTURAL BASIS FOR THE INTERFERENCE BETWEEN NICORANDIL AND SULFONYLUREA ACTION" DIABETES, NEW YORK, NY, US, vol. 50, no. 10, October 2001 (2001-10), pages 2253-2259, XP008007177 ISSN: 0012-1797
- SEINO SUSUMU ET AL: "Physiological and pathophysiological roles of ATP-sensitive K+ channels." PROGRESS IN BIOPHYSICS AND MOLECULAR BIOLOGY. FEB 2003, vol. 81, no. 2, February 2003 (2003-02), pages 133-176, XP002344317 ISSN: 0079-6107

## Description

The invention concerns methods for identifying an increase in risk for angina pectoris as well as probes, primers, polypeptides or polynucleotides suitable for said methods.

In the western world, cardiovascular diseases are the leading cause of death among both sexes, and coronary heart disease, especially coronary artery disease is the major cause of cardiovascular diseases. Angina, also called angina pectoris, is temporary chest pain or a sensation of pressure that occurs while heart muscle is not receiving enough oxygen. When the arteries are narrowed or blocked so that blood flow to the muscle cannot increase to meet the need for more oxygen, ischemia may occur, resulting in pain (i.e. angina).

Usually angina pectoris results from coronary artery disease, but it can also result from other coronary heart diseases. Not everyone with ischemia experiences angina pectoris. Ischemia without angina is called silent ischemia. The danger of silent ischemia lies in the fact, that the heart tissue is undergoing damage as well, without the patient noticing it. Thus, a possible damage of the heart is often not recognized by the patient or the physician in charge until it ultimately results in a myocardial infarction. Therefore, there is great need of diagnostic principles for recognizing vascular and especially coronary degenerations (in the following referred to as coronary heart disease) enabling an early diagnosis and thus early therapeutic or preventive measures.

Thus, it is the object of the invention to provide improved methods for the diagnosis and treatment of coronary heart disease, esp. angina pectoris, in diabetes patients.

This is achieved by a method of identifying an increase in risk for coronary heart disease in a diabetes patients, wherein the presence of another amino acid than Glutaminic Acid at position 23 and / or the presence of another amino acid than Valine at position 337 in the Kir6.2 protein is determined in a sample.

In the following, standard abbreviations for nucleotides and amino acids (such as the three or one letter code of amino acids) are used synonymously with the full-length terms.

The identification of the polymorphism in the protein sequence of Kir6.2 comprising the amino acid exchange at position 23 (especially if from Glu to Lys) and/or the amino acid exchange at position 337 (especially if from Val to Ile) in the Kir6.2 protein can be used to predict an increased or normal risk for angina pectoris. It can be used, e.g. in
1) methods based on sequencing the protein region of interest (e.g. standard protein degradation, analysis of protein sequence fragments with mass spectrometry);
2) methods based on using anti-Kir6.2 antibodies against the region of interest (e.g. ELISA);
3) methods based on analyzing functional activity of Kir6.2 in in-vitro assays using e. g. human, animal, bacterial, or yeast cells.

The present invention further relates to a method of identifying an increase in risk for coronary heart disease in an individual, wherein the presence of a variation in the nucleotide sequence on one or both (preferably both) alleles of the Kir6.2 gene leading to a Kir6.2 polypeptide sequence harbouring another amino acid than Glutaminic Acid at 23 and/or another amino acid than Valine at position 337 in the Kir6.2 protein is determined in a sample.

The identification of the polymorphism in the nucleotide sequence on one or both alleles of the Kir6.2 gene leading to the amino acid exchange at position 23 (especially if from Glu to Lys) and/or at position 337 (especially if from Val to Ile) in the Kir6.2 protein can be used to predict increased risk for angina pectoris, in diabetes patients.

It can be used e.g. in
1) methods based on sequencing the nucleotide region of interest (e.g. pyrosequencing, sequencing methods using radio-labeled nucleotides, or nucleotides which are labeled with a fluorescent dye, analysis of sequence fragments with mass spectrometry);
2) methods based on the hybridization of nucleotide sequences to the region of interest (e.g. DNA microarrays);
3) methods based on analyzing amplification products of the nucleotide region of interest (e.g. TaqMan^{™} analysis).

The K⁺ inwardly rectifying channel Kir6.2 (KCNJ11 or Bir) is a subunit of the pancreatic islet ATP-sensitive potassium channel (I_{KATP}). The gene for Kir6.2 has been mapped to chromosome 11p15.1 and the Kir6.2 protein consists of 390 amino acids. Because of its key role in glucose-stimulated insulin secretion, Kir6.2 is assumed to be a candidate gene for genetic defects involved in the outset of type II diabetes mellitus (Yamada et al. (2001) Diabetes Metab Res Rev 17: 213-216).

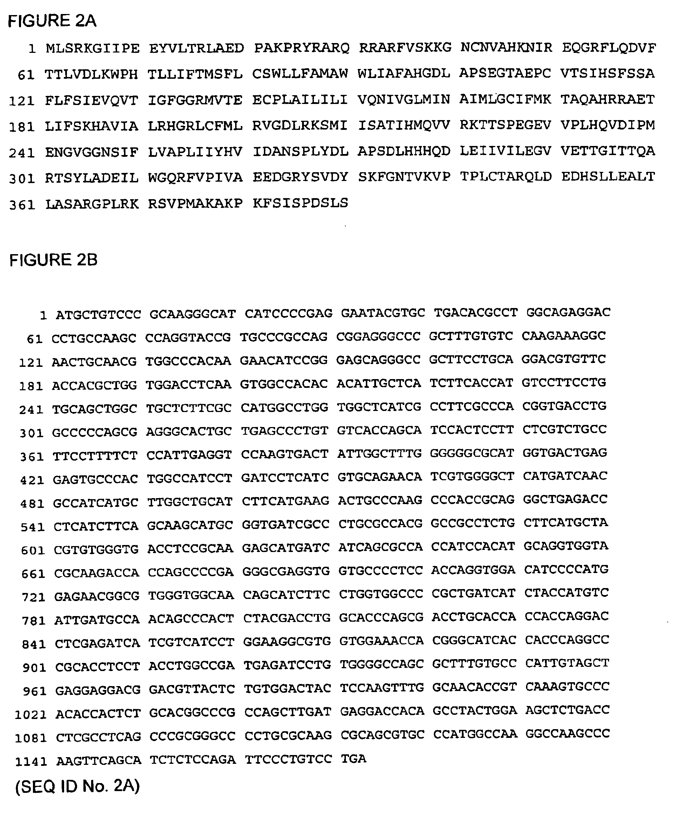

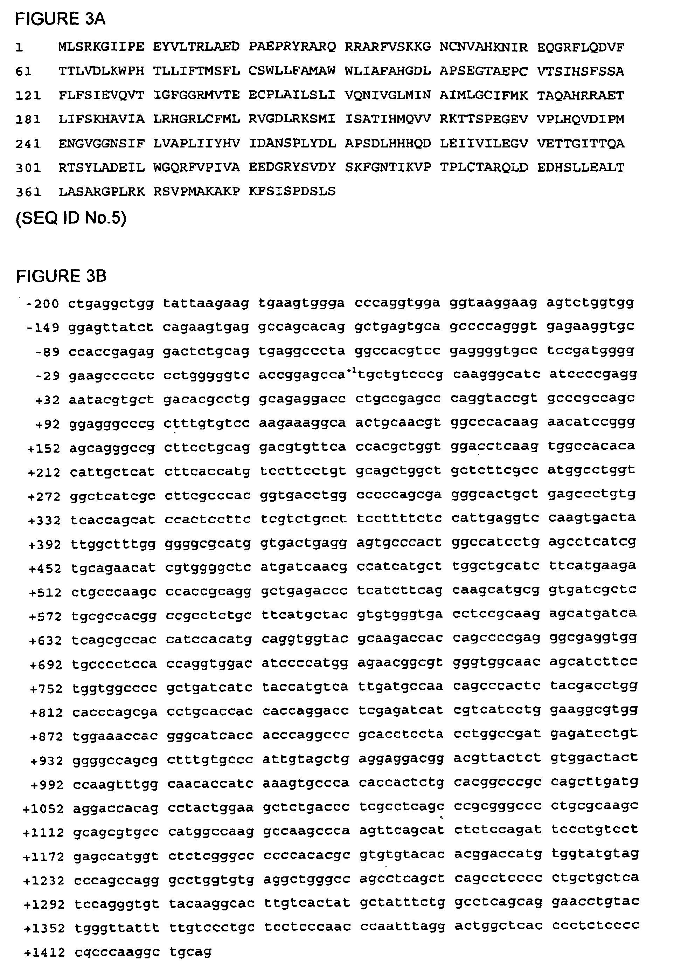

Several protein, genomic and coding polynucleotide sequences of Kir6.2 are known in the state of the art. E.g. several human protein / genomic DNA / coding sequences are publicly available from the NCBI (National Center for Biotechnology Information; National Library of Medicine, Building 38A, Bethesda, MD 20894, USA; www.ncbi.nhm.nih.gov) data base under the accession numbers XP006398 (Seq. ID No. 3), (Seq. ID No. 5) (protein sequence) XM006398 (Seq. ID No. 4), (Seq. ID No. 6) (coding sequence). One genomic Kir6.2 sequence is publically available from a genomic contiguous sequence that is published under the NCBI accession number NT_009307 (homo sapiens chromosome 11 genomic contig.; size: 8665930 bp). The alignment of the mRNA/coding/genomic Kir6.2 sequences is publicly available using the following internet link:
hftp://www.ncbi.nim.nih.gov/entrez/viewer.fcgi?SendTo=on&db=nucleotide&dispmax=1 &extrafeat=-1&list_uids=NT_009307.12&showndispmax=1 &view=graph&_from=5656500&_to=565 9500&_sfrom=5657000&_font=default&_Ilen=60&_slen=4000.
As can be gained from such an alignment, the sequence stretch from position 5656500 to 5659500 (SEQ ID No. 13) comprises the genomic Kir6.2 locus. As shown in figures 2 and 3, the different Kir6.2 sequences differ in positions 23 and/or 337 with respect to the protein sequence and with respect to the according positions of the coding sequence (67/68/69 and 1009/1010/1011, respectively). Since the coding sequence stems from transcription products of genomic DNA, several different variants of the genomic sequences with respect to positions 5657106/107/108 and 5657478/79/80 are bound to exist, too.

The term Kir6.2 refers to Kir6.2 protein and nucleic acid. The term protein comprises polypeptides as well as proteins consisting of more than one polypeptide chain, e.g. polypeptides linked to each other by means of known bonds (e.g. S-S bridges) to form one protein unit. The term Kir6.2 protein can refer to the any Kir6.2 protein or polypeptide or functional fragment thereof. A Kir6.2 fragment can e.g. be any Kir6.2 protein or nucleic acid that is shorter than the Kir6.2 proteins or nucleic acids having sequences according to one of the SEQ IDs 1 to 8 or 13. Kir6.2 fragments are preferably functional fragments of Kir6.2. A functional fragment of a Kir6.2 protein is a protein fragment having at least one of the functions of Kir6.2, see e.g. above. A functional nucleic acid of Kir6.2 is a Kir6.2 nucleic acid fragment that e.g. encodes for Kir6.2 protein or a functional fragment thereof or that is able to specifically hybridize with a Kir 6.2 nucleic acid.

The terms protein sequence, amino acid sequence and polypeptide sequence are used synonymously in the context of this application.

Surprisingly, studies of the inventors revealed that the most frequent variant at positon 23 of the Kir6.2 polypeptide is a Glu (Glutaminic Acid, E). Moreover, they revealed that the most frequent variant at position 337 is a Val (Valine, V). Thus, these amino acids at the respective positions are regarded as the Kir6.2 "wild type" within the scope of this application. Thus, if it is referred to exchanges or mutations or less frequent variants in the amino acid sequence in the context of this application, deviations from the wild type amino acids at either position 23 and/or 337 from this "wild type" are meant.

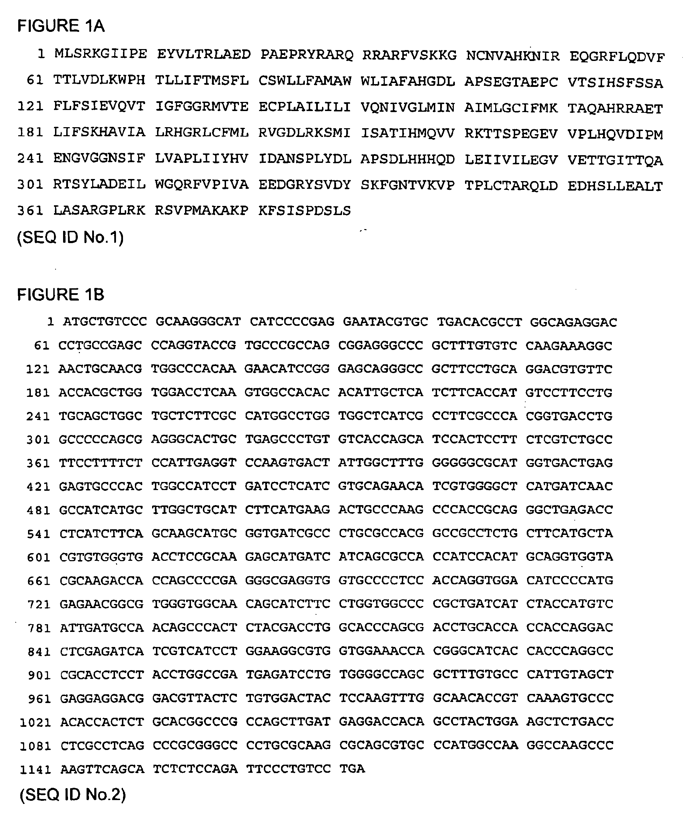

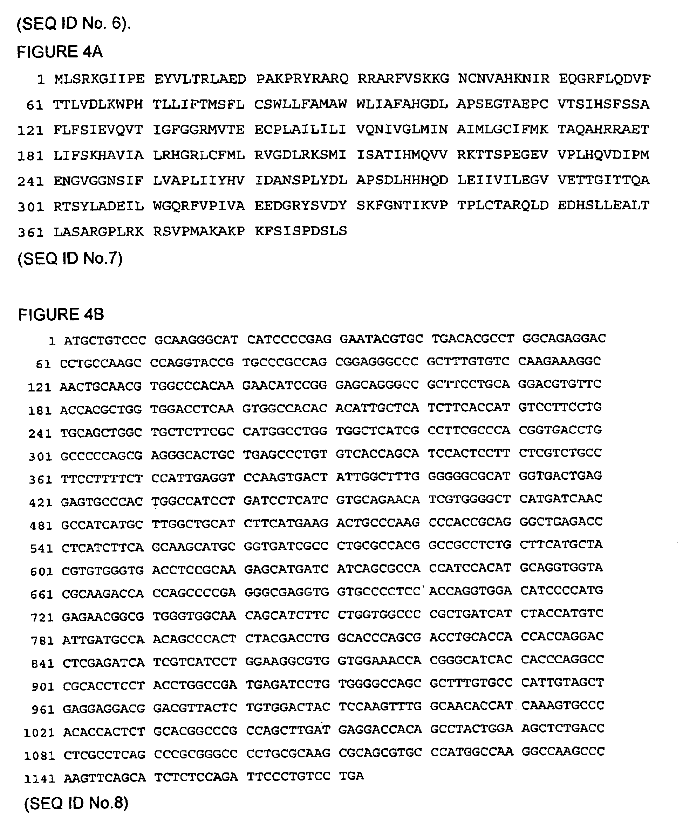

The sequences according to SEQ ID No. 1 (NCBI accession No. D50582; see Fig. 1A) and 2 (NCBI accession No. D50582; see Fig. 1B) represent the Kir6.2 wild type with respect to position 23 and 337 of the polypeptide sequence (and respectively, positions 67/68/69 or position 1009/1010/1011 of the coding and positions 5657106/07/08 and 5657478/79/80 of the genomic sequence). The sequences SEQ ID No. 3 (NCBI accession No. XP 006398; Fig. 2A), SEQ ID No. 4 (NCBI accession No. XM006398, Fig. 2B) derived from the NCBI database, and the novel sequences SEQ ID No. 5 and SEQ ID No. 6 on the other hand, each harbour one less frequently occurring amino acid at either position 23 or position 337 of the polypeptide sequence (and respective sequence variations at position 67 or position 1009 of the nucleotide sequence). The novel sequences SEQ ID No. 7 (Fig. 4A) and 8 (Fig. 4B) harbour less frequently occurring variants at both positions 23 and 337 of the amino acid chain: A Lysine at position 23 (with an AAG at positions 67/68/69 of the coding sequence) and an Isoleucine at position 337 of the amino acid sequence (with an ATC at positions 1009/1010/1011 of the coding sequence).

Single nucleotide polymorphisms (SNPs) represent variants of a given nucleotide sequence harbouring exchanges at single nucleotide positions; SNPs are well known in the art.

Several single nucleotide polymorphisms (SNP) have been identified in the Kir6.2 gene leading to amino acid exchanges in the translated protein, such as E23K (exchange from Glutaminic acid to Lysine at position 23 of the protein), L270V (exchange from Leucine to Valine at position 270) and V3771.
In a recent study Kir6.2 variant E23K has been linked to type II diabetes mellitus in a Caucasian population (Hani et al. (1998) Diabetologia 41: 1511-1515).

Surprisingly, studies of the inventors identified for the first time a close correlation between the presence of mutations within the amino acid sequences at positions 23 and/or 337 of the Kir6.2 protein and a predisposition for angina pectoris.

The detection of genetic polymorphisms in the Kir6.2 gene, in particular Kir6.2-23-KK (Kir6.2 variants having Lysine (K) at position 23 in the protein as a consequence of polymorphisms at the corresponding position on both alleles of the Kir6.2 gene) and Kir6.2-337-II (Kir6.2 variants having Isoleucine (I) at position 337 in the protein as a consequence of polymorphisms at the corresponding position on both alleles of the Kir6.2 gene), by analyzing human DNA or Kir6.2 protein can be used e.g. (a) as genetic markers for preventive treatments of coronary heart disease, more specifically coronary artery disease and yet more specifically angina pectoris, especially in diabetes patients, (b) as a genetic marker for the adaptation of drug dose, (c) as a genetic marker for drug screening set-up adaptation and (d) as a genetic marker for patient selection in phase/clinical studies.

By allowing for an early identification of a predisposition for coronary heart disease, the method according to the invention allows for an early preventive or curative treatment of the diabetes patient before symptoms such as angina, or damages to the heart tissue occur: Identification of one or both of the above amino acid exchanges or their underlying nucleotides on mRNA or genomic level gives a clear indication for the treating physician to screen for an already persisting damage to coronary heart tissue or vessels, or to prescribe preventive pharmaceuticals even before damage and/or pain occur.

Moreover, the surprising correlation between said amino acid exchanges and the onset of coronary heart disease allows for a more effective treatment of coronary heart disease by hinting to the necessity of a higher dosage for certain pharmaceuticals or indicating the necessity of a certain/different type of medication when compared to coronary heart disease patients that do not exhibit said Kir6.2 amino acid exchanges.

Thus, another embodiment of the invention concerns a method of adapting the dosage of a pharmaceutical useful for treating and/or preventing angina pectoris, wherein the presence of a Kir6.2 with
a) an amino acid other than Glutaminic Acid at position 23 and/or an amino acid other than Valine at position 337 in the Kir6.2 protein,
b) a variation in the nucleotide sequence on one or both alleles of the Kir6.2 gene leading to amino acid other than Glutaminic Acid at position 23 and/or other than Valine at position 337 in the Kir6.2 protein or
c) Kir6.2-23-KK and/or Kir6.2-337-II is determined in a sample of a diabetic patient.

The selection of individuals concerns diabetes patients; the pharmaceutical is preferably a pharmaceutical for the prevention or treatment of angina pectoris. Preferably, the amino acid exchange at position 23 is from Glu to Lys and/or the amino acid exchange at position 337 is from Val to Ile. The sample is preferably an isolated protein or nucleic acid, a biological sample, such as a histological sample, a cell or tissue extract or a cell.

A wide panoply of pharmaceuticals for the treatment or prevention of coronary heart disease or angina pectoris is known in the state of the art.

Since present studies for the first time identified Kir6.2 as being involved in the processes underlying or correlating with coronary heart disease, a further aspect of present invention concerns the use of Kir 6.2 for the identification of pharmaceuticals useful for treating and/or preventing coronary heart disease.

The term "pharmaceutical" refers to any substance or combination of substances that can be used for the curative or preventive treatment of a disease state in an individual. A substance can be any biological or chemical substance or natural product extract, either purified, partially purified, synthesized or manufactured by means of biochemical or molecular biological methods.

A pharmaceutical considered as being useful or active in preventing or treating a coronary heart disease in the sense of the different aspects of present invention can be any substance or combination of substances having an influence of one of the functions of Kir6.2 or on the amount of Kir6.2 (protein or nucleic acid) in a cell or on Kir6.2 expression.

To this end, the substance can modulate any of the functions of Kir6.2 (e.g. those as listed above). Kir6.2 protein activity can be modulated by the substance e.g by direct interaction and interference of Kir6.2 polypeptide/protein or fragments thereof. The substance can also modulate Kir6.2 expression, e.g. on the level of transcription (initiation, elongation, processing, etc), transcript stability, translation. Moreover it can modulate the posttranslational modification, protein folding etc. of Kir6.2. The substance can exert the above effects directly or indirectly (indirectly meaning i.e. by interfering (positively or negatively) with natural signalling cascades having influence on Kir6.2 function / protein activity / expression etc.)

Another embodiment of present invention concerns a method of screening for pharmaceuticals useful for treating and/or preventing angina pectoris comprising the steps
a. Providing two samples containing Kir6.2,
b. Contacting one sample with a potential pharmaceutical,
c. Determining Kir6.2 activity or Kir6.2 structure in the presence and in the absence of the potential pharmaceutical,
d. Comparing the Kir6.2 activity in the presence with that in the absence of the potential pharmaceutical.

The activity or structure (i.e. the folding of Kir6.2 protein) in presence of the potential pharmaceutical substance can also be compared to the activity or structure of the wild type Kir6.2 protein.

Preferably, the sample contains or is an isolated protein or protein fragment of Kir6.2 or an isolated nucleic acid or nucleic acid fragment of Kir6.2.

In the context of this invention the term "isolated" with respect to "isolated protein", "isolated nucleic acid" and so on, refers to proteins/nucleic acids purified from natural sources as well as recombinant proteins/nucleic acids (which, of course, can also be purified).

Since the amino acid exchanges within Kir6.2 identified to correlate with coronary heart disease are very likely to affect the treatment and/or prevention of coronary heart disease, the Kir 6.2 used for one of the above uses or methods can e.g. be
a) a Kir 6.2 protein or fragment thereof containing an amino acid other than Glutaminic Acid at position 23 and/or an amino acid other than Valine at position 337 in the Kir6.2 protein,
b) a Kir 6.2 nucleic acid or fragment thereof containing a variation in the nucleotide sequence on one or both alleles of the Kir6.2 gene leading to amino acid other than Glutaminic Acid at position 23 and/or other than Valine at position 337 in the Kir6.2 protein or
c) Kir6.2-23-KK and/or Kir6.2-337-II.

In screening for the ability of a potential pharmaceutical to restore protein function and/or folding of a Kir6.2 harbouring one or both of said amino acid exchanges to that of the wild type protein, pharmaceutically active substances for the treatment and/or prevention of coronary heart disease can be identified.

Pharmaceutical screening methods and systems especially if used in a high throughput format using isolated protein or protein fragments, cells expressing said protein, etc. are well known in the state of the art.

Suitable analytical methods or analytical systems, so-called assays which are used to measure the activity or concentration of defined target molecules, in the context of the present invention, the activity or folding of Kir6.2 (so-called targets, mostly proteins or nucleic acids), as a parameter for the effectiveness of a potential pharmaceutical compound, are well known in the state of the art. Assays can e.g. be biochemical analytical methods or systems using isolated or partly isolated components that are put together to a reaction mixture within a defined space and time, in which the effectiveness of the potential pharmaceutical compounds can be tested. Another example of assays comprise biochemical analytical methods or systems , in which the activity of the target molecule and the effectiveness of a potential to influence this activity, can be determined within a cell.

An assay is any type of analytical method or system to monitor a biological process. Suitably, molecular cascades and mechanisms representing parts of physiological metabolic pathways but also of pathological conditions are reproduced in cellular or biochemical (in vitro) systems. The pharmacological activity of a potential pharmaceutical compound can thus be determined according to its capability of interfering with or modulating these cascades or mechanisms.

For the use in drug screening, especially the high throughput screening for novel pharmaceutical compounds, the assay needs to be reproducible and is preferably also scalable and robust. The assay is preferably suitable for high throughput screening of chemical substances for their ability of modulating the activity of the target molecule under investigation. The type of assay depends e.g. on the type of target molecule used (e.g. polypeptide or polynucleotide) and the "read out", i.e. the parameter, according to which the activity of the target molecule is determined (see below).

Different types of such assays are commonly known in the state of the art and commercially available from commercial suppliers.

Suitable assays for different purposes encompass radioisotopic or fluorescent assays, for example fluorescence polarization assays (such as those offered commercially by Panvera) or Packard BioScience (HTRF; ALPHAScreen^{™}) for measuring the interaction of a labeled member with a non-labeled member (e.g. the interaction of labeled protein receptors with their unlabeled ligands).

More examples include cell-based assays, wherein a cell line stably (inducibly or not; chromosomal or episomal) or transiently expresses a recombinant protein of interest. These assays comprise e.g. reporter gene assays, wherein the regulation of a certain promotor or a signal transduction pathway of a member of a signal transduction cascade is measured according to the activity of a reporter enzyme, the expression of which is under the control of said certain promotor. For this type of assay, a recombinant cell line has to be constructed containing the reporter gene under the control of a defined promotor that is to be investigated itself or that is regulated by the signaling cascade under investigation. Suitable reporter enzymes are commonly known within the state of the art and comprise firefly luciferase, renilla luciferase (e.g. commercially available by Packard reagents), ß-Galactosidase. Suitable cell lines depend on the aim of the assay but comprise mostly cell lines that are easy to transfect and easy to cultivate, such as, e.g. HeLA, COS, CHO, NIH-3T3, etc.

Assays for measuring the intracellular ion level comprise e.g. FLIPR (fluorometric imaging plate reader, commercially available from Molecular Devices) assays, wherein an argon laser light source combined with a cooled CCD camera allows for parallel measurements in 384 well plates transient ion signals (such as Ca2⁺, etc) within cells (e.g. neuronal cells or other cells (e.g. cells recombinantly or naturally expressing certain ion channels). FLIPR assays allow e.g. for monitoring of intracellular calcium using certain fluorochromes, such as Fluo-3, Fluo-4, or monitoring intracellular pH using BCECF or BCPCF pr specific FLIPR assay kits, or detecting membrane potential changes using e.g. DiBAC or specific FLIPR assay kits, or monitoring of membrane polarization. For the monitoring of other intracellular ions, e.g. zinc or sodium, other dyes known in the state of the art can be used. Other types of assays and other types of read outs are commonly known to persons with skills in the art.

For the determination of ion channel activity such as that of Kir6.2 (which control e.g. intracellular ion concentrations and can thus be employed for measurement of intracellular ion concentrations) e.g. membrane potential sensitive assays and dyes can be used such as DiBAC or Molecular Devices' membrane potential assay kit on FLIPR technology; mitochondrial membrane polarization measuring JC-1 dye with FLIPR technology; ion sensitive dyes, etc. Assays based on patch-clamping or atomic adsorption spectroscopy-based Rubidium ion efflux measurement are especially suitable for determining of intracellular potassium concentrations, and so on. Further automatical devices and for detecting certain changes and states within cells are known to the person of skill in the art and comprise, e.g. the Acumen detector (flurescence-based laser scanning reader that allows for 3dimensional reconstitution of distribution of suitably labeled objects) by ACUMEN bioscience. Other types of assays and other types of "read out" are well known in the state of the art.

For the above screening method or use, it is preferred if the Kir 6.2 protein comprises or has the sequence according to SEQ ID No. 3, 5 or 7. It is also preferred, if the protein fragment according to the above method or usecomprises or has the sequence according to SEQ ID No. 3, 5 or 7. The nucleic acid according to the above screening method preferably comprises or has the sequence according to SEQ ID No. 4, 6 or 8. And the nucleic acid fragment according to said screening method preferably comprises or has the sequence according to SEQ ID 4, 6 or 8.

It is very likely that all amino acid exchanges at position 23 from the wild type variant Glu and/or at position 337 from the wild type variant Val of Kir6.2 affect the predisposition to coronary heart disease. Thus, the amino acid exchange from the wild type basically can be of any type. It is preferred, if the amino acid exchanges at positions 23 and/or 337 refer to the incorporation of amino acids with properties different from those of the wild type amino acids Glu23 and Val337, respectively. Thus, is preferred, if a nonpolar or basic amino acid is placed at position 23 instead of the acidic Glutaminic Acid. It is even more preferred, if it is a basic amino acid, especially Lysine (Lys, K). With respect to position 337, which in the wild type polypeptide carries the nonpolar amino acid Valine (Val, V), it is preferred, if a nonpolar amino acid with a side chain that is more bulky than that of Valine, a basic or acidic amino acid is placed at position 337. More preferably, a nonpolar amino acid with a side chain that is more bulky than that of Valine is placed at position 337 within the scope of this invention. Most preferred embodiments of the above methods comprise variants carrying a Lysine at position 23 of the Kir6.2 and / or an Isoleucine at position 337 of the Kir6.2.

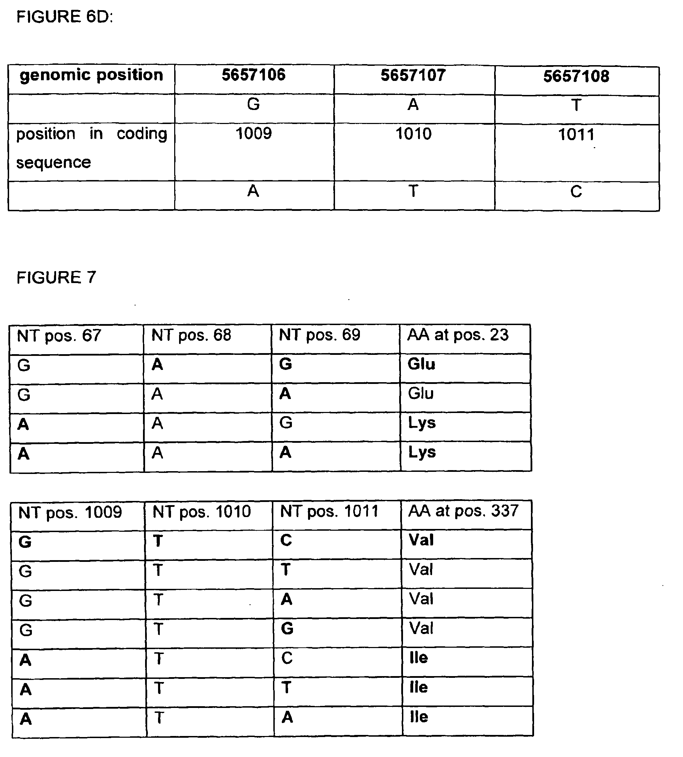

Due to the wobble of the genetic code, there are several possibilities of nucleotide exchanges resulting in the above amino acid exchanges. The genetic code is well known (for reference, see also Alberts et al., Molecular Biology of the Cell, 3^{rd} edition). With respect to amino acid position 23 in the polypeptide, the exchange from Glutamic Acid to another amino acid can be caused by a mutation of one or more of the positions 67/68/69 of the coding sequence according to SEQ. ID No. 1. With respect to amino acid position 337, the exchange from Valine to another amino acid can be caused by a mutation of one or both of the position 1009 or 1010 of the DNA sequence according to sEQ - ID No. 1. Since, as stated above, certain types of amino acid exchanges are preferred, preferred with respect to the nucleotide sequence are also those nucleotide exchanges resulting in said preferred amino acid exchanges. Even more preferred are those nucleotide exchanges that lead to an E23K (exchange from Glutaminic Acid to Lysine at position 23 of the polypeptide sequence) and/or V337I exchange (exchange from Valine to Isoleucine at position 337; see also figure 7). The most preferred SNPs are G67A (exchange from G to A at position 67 of the coding sequence) and/or G1009A (exchange from G to A at position 1009 of the coding sequence). Preferred embodiments encompass also exchanges of the respective nucleotides within the genomic sequence affecting the amino acid sequence at positions 23 and / or 337 within the Kir6.2 protein.

According to preferred embodiments of the different aspects of present invention, the individual is a diabetic patient; and it is even more preferred if the diabetes is diabetes type II (diabetes mellitus). It is also preferred if the coronary heart disease is angina pectoris.

It is advantageous, if the amino acid exchange at position 23 is from Glu to Lys and/or if the amino acid exchange at position 337 is from Val to Ile. The sample is preferably an isolated protein or nucleic acid, a histological sample, a cell or tissue extract or a cell. It is also preferred, if the sample is isolated from the human body.

Another preferred embodiment of the invention refers to one of the above methods, wherein the presence of Kir6.2-23-KK and/or Kir6.2-337-II is determined in the sample. The sample can e.g. be a biological, such as a histological sample, a cell or tissue extract or a cell, and the sample is preferably isolated from the human body.

Biological material and biological samples comprise, e.g. cells, preparations or parts of tissue or organs (e.g. brain, blood, liver, spleen, kidney, heart, blood vessels, etc.), preferably if derived from a vertebrate, and more preferably from a mammal including a human. Comprised are also cells from a cell culture, preferably a eukaryotic cell culture comprising cells derived either from multicellular organisms and tissue (such as HeLA, CHO, COS, SF9 or 3T3 cells) or single cell organisms such as yeast (e.g. s. pombe or s. cerevisiae), or a procaryotic cell culture, preferably Pichia or E.coli. Cells and samples derived from tissue can be gained by well-known techniques, such as taking of blood, tissue punction or surgical techniques. The preparation of recombinant molecules and the purification of naturally occuring molecules such as DNA or proteins from cells or tissue, as well as the preparation of cell- or tissue extracts is well known to the person of skill in the art (see e.g. also the standard literature listed below).

The presence of the amino acid exchange can e.g. be determined by analyzing the genomic DNA of the individual. Suitable techniques are well known in the art and comprise, e.g.: Different PCR techniques, chip hybridization, slot / dot or Southern blotting.

PCR (polymerase chain reaction) is an in vitro technique that enables the specific amplification of sequence stretches having nucleotide stretches of known sequence in their 5'and 3'vicinity. In order to amplify a given sequence, it is sufficient, if the sequence in the 5' region of the sequence to be amplified is known. In this case, a fragment of the polynucleotide to be amplified is to be generated first (this can be done by known techniques, such as digestion with a restriction endonuclease). Next, a DNA-molecule of known sequence (a "linker") is coupled to the 3'-end of the generated polynucleotide fragment by means of a ligase (such as T4 DNA ligase, which is commercially available from different suppliers). The resulting sequence is thus surrounded by two known sequences, the known 5'-sequence and 3'the known linker sequence, enabling the specific amplification by PCR (in this case a linker-mediated PCR "ImPCR").

For amplifying the sequence of choice, short single-stranded DNA molecules ("primers") are used, which are complementary to the sequence stretches framing the polynucleotide sequence to be amplified. The polynucleotide template can either be DNA or RNA. The primers are then annealed to the single stranded template and elongated, under defined and well known conditions, by specific enzymes, the so called polymerases (either DNA polymerases recognising DNA as template and producing complementary DNA polynucleotides or reverse transcriptases, recognising RNA as template and producing complementary DNA polynucleotides), thus leading to the generation of new DNA strands having a sequence complementary to that of the template strand. By chosing defined sequences of incubation steps at defined temperatures and of defined time intervalls, that are repeated periodically, a sequence of denaturation / annealing / polymerisation steps is generated that ultimately leads to the exponential amplification of the polynucleotide of interest. In order to be able to apply the necessary temperatures for denaturation without destroying the polymerase, heat-stable enzymes, well tolerating temperatures as high as 95°C and more, such as Taq-DNA polymerase (DNA polymerase from thermus aquaticus), PFU etc, both commercially available from different suppliers, are used. The choice of suitable polymerases depends on the purpose of use (e.g. for cloning by PCR, polymerases with proofreading capabilities, such as PFU are preferably chosen) and belongs to the skills of the person of the art.

A typical PCR reaction comprises the polynucleotide template (e.g. 0,01 to 20 ng), two suitable, primers (in a concentration of e.g. 0,2 to 2 µM each), dNTPs (in a concentration of e.g. 200µM each), 1 to 2mM MgCl2 and 1 to 10 units of a heat-stable polymerase, such as Taq. Typical components and buffers are well known to the person of skill in the art and commonly available by commercial suppliers.

Suitable primers can be generated by means of chemical synthesis according to well known protocols. Such primers are also commercially available by different suppliers, such as MWG Biotech etc.

DNA and RNA templates, also cDNA templates can be generated by means of well known standard procedures (see, e.g. the below standard literature) and can also be purchased from commercial suppliers, such as Promega and Stratagene, etc.

Methods for the preparation of biological samples containing genomic DNA (and / or RNA and / or protein) are well known in the art. For reference, see e.g. Sambrook et al., or Current Protocols in Molecular Biology or Protein Science.

According to a preferred embodiment of the invention, the presence of the amino acid exchange is determined by a method consisting of or comprising the following steps:
e. Preparing from an individual a biological sample containing genomic DNA.
f. If the target sequence is not to be directly analysed by means of in situ methods, such as in situ PCR or direct genomic sequencing directly using the above sample (e.g. a tissue sample or cells), the genomic DNA from the sample according to a) has to be isolated or at least partially purified.
g. Amplification of a polynucleotide fragment by means of a PCR reaction using primers able to amplify a polynucleotide fragment comprising those nucleotide positions of the genomic kir 6.2 sequence that correspond to positions 67/68/69 and/or 1009/1010/1011 of the coding sequence.
h. Sequencing of the polynucleotide fragment according to c).

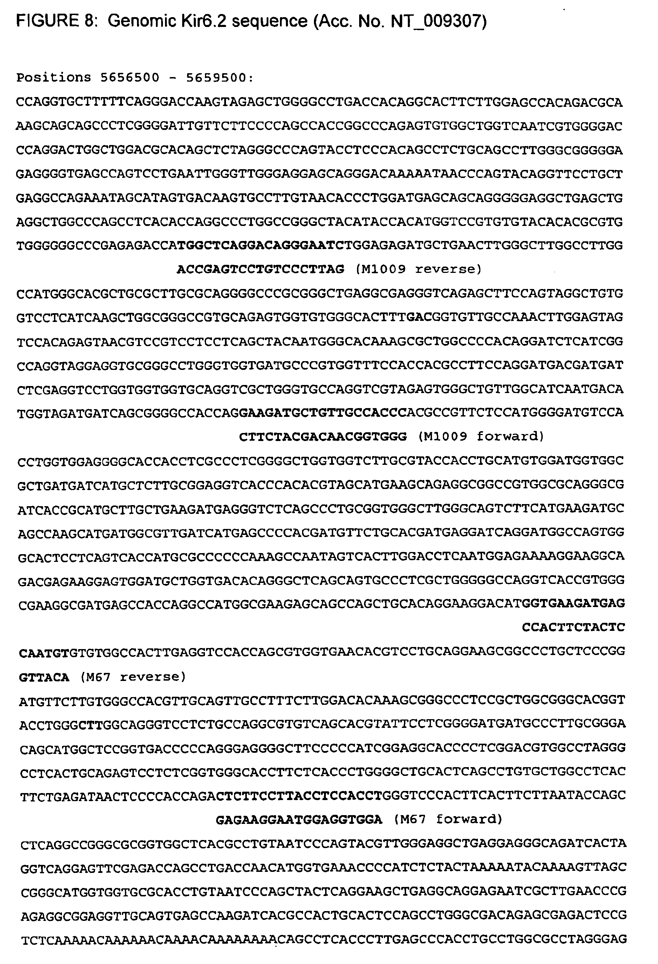

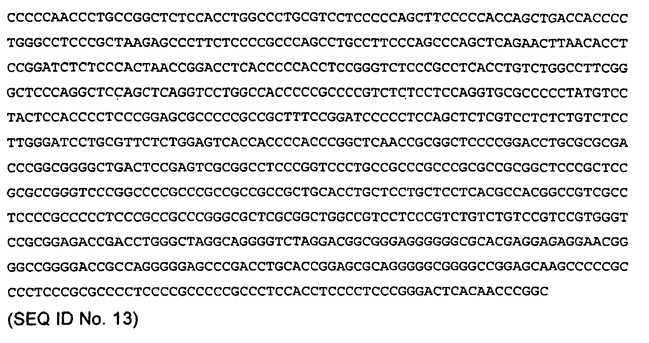

With respect to the genomic Kir6.2 sequence according to SEQ ID No. 13 the above "corresponding positions" within the genomic sequence are 5657106/5657107/5657108 and 5657978/5657979/5657980, respectively (see figure 8).

The presence of one or more nucleotide exchanges in the genomic sequence leading to one or more of the above amino acid exchanges in the protein can then be identified according to known standard procedures (e.g. using labeled sequencing primers and performing autoradiographical or fluorometric detection of the signals).

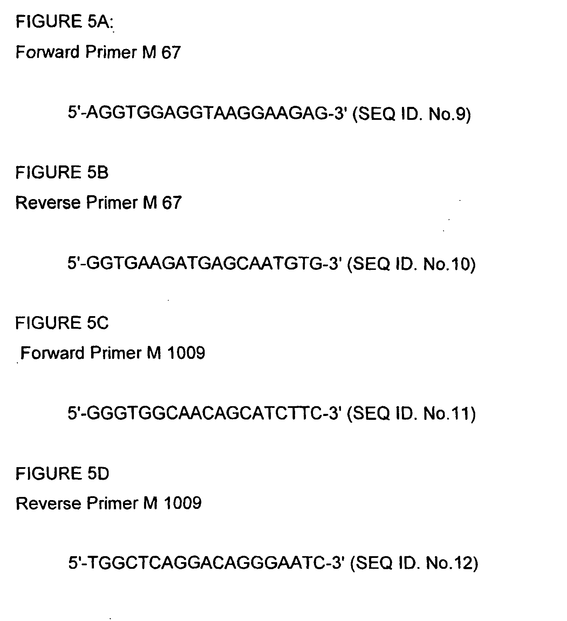

The selection and design of suitable primers for the amplification of the above polynucleotide fragment belongs to the abilities of the person with skills in the art. For reference, see for example Sambrook et al., or Current Protocols in Molecular Biology. The same holds true for the preparation of said primers, which can also be ordered from commercial suppliers (such as MetaBion, Martinsried, Germany). Preferably, at least one of the primers according to SEQ. ID No. 9, 10, 11 or 12 is used (see Fig. 5) for sequencing and/or amplification.

According to another preferred embodiment of the present invention, the presence of the amino acid exchange is determined by a method comprising or consisting of the following steps:
a. Preparing a biological sample containing chromosomal / genomic DNA.
b. Isolating the chromosomal DNA from the sample according to a).
c. Immobilizing it on a carrier.
d. Hybridizing to the immobilized DNA one or more probes able to bind to the Kir6.2 Sequence harbouring one or more nucleotide exchanges leading to one or both of said amino acid exchanges with higher affinity than to the wild type Kir6.2 Sequence under stringent conditions.

The hybridization can be visualized according to standard methods such as autoradiography or fluorescence using probes labeled accordingly.

The presence or absence of a nucleotide exchange in the genomic Kir6.2 Sequence leading to an amino acid exchange at position 337 and/or 23 in the Kir6.2 protein can then be identified from comparing the hybridization patterns and / or intensities stemming from the patient samples when compared to the hybridization pattern and / or intensities of control samples containing wild type genomic DNA.

Isolated polynucleotides and oligonucleotides can be used for hybridizing at different conditions of stringency.

Stringency describes reaction conditions that influence the specificity of hybridisation or annealing of two single stranded nucleic acid molecules. Stringency, and thus specificity of a reaction depends, inter alia, of the temperature and buffer-conditions used for a reaction: Stringency, and thus specificity, can e.g. be increased by increasing the reaction temperature and/or lowering the ion strength of the reaction-buffer. Conditions of low stringence (and thus low reaction and hybridisation specificity) exist for example, if a hybridisation is performed at room temperature in 2xSSC-solution. Conditions of high stringency comprise e.g. a hybridisation reaction at 68°C in 0,1xSSC and 0,1% SDS solution.

Hybridisation under conditions of stringency within the different aspects of present invention is preferably understood to be:
1) Hybridising a labelled probe with a nucleic acid sample to be analysed at 65°C, or in the case of oligonucleotide probes, at 5°C below the annealing or melting temperature of the duplex consisting of oligonucleotide and sample (annealing and melting temperature are in the following understood to be synonyms) over night in 50mM Tris pH 7,5, 1M Nacl, 1% SDS, 10% Dextran Sulfate, 0,5 mg/ml denatured salmon or hering sperm DNA.
2) Washing for 10 minutes in 2xSSC at room temperature.
3) Washing for 30 minutes in 1xSSC/0,1%SDS at 65°C (or in the case of oligonucleotides: 5°C below the annealing temperature).
4) Washing for 30 minutes in 0.1xSSC/0,1%SDS at 65°C (or in the case of oligonucleotides: 5°C below the annealing temperature).

Oligonucleotides are polynucleotide and preferably DNA-fragments having a length of 15 to 30, preferably 20 nucleotides. The annealing temperature is determined according to the formula Tm=2x (number of A+T) + 4x (number of G+C)°C.

For preparing a 2xSSC or a 0,1xSSC (or any other kind of SSC dilution), e.g. a 20x SSC solution is diluted accordingly. 20xSSC consists of 3M NaCl/0,3 M Na-Citrate x2H₂O.

Before performing of a hybridisation reaction, the polynucleotides are, if wanted after performing electrophoretic separation (then: Southern Blot (DNA) or Northern Blot (RNA)) or without electrophoretic separation (then: slot or dot Blot), transferred to a suitable membrane, e.g. a nylon or nitrocellulose membrane. Hybridisauion is performed using a suitably labelled probe. Suitable labelling techniques are e.g. radioactive labelling or labelling using fluorescence dyes. The probe is a single stranded polyribo- or polydesoxyribonucleotide being single stranded naturally or being usually double stranded and having been made single stranded by denaturation. This probe binds to the DNA or RNA sample (which is also in single stranded state) by means of base pairing.

The DNA can e.g. be directly be immobilized on a chip matrix (such as e.g. Affymetrix Chips, etc.) or on a suitable membrane (e.g. Nitrocellulose, Nylon or others suitable for dot or slot blots) or it can be transferred to a gel matrix, separated electrophoretically and blotted to a suitable membrane (e.g. Nitrocellulose, Nylon or others suitable for Southern Blots) afterwards. Suitable chips or membranes, as well as according methods are well known in the state of the art. The above methods, such as Chip Hybridization, Dot / Slot or Southern Blotting, are well known in the art (for reference, it is e.g. referred to the literature listed below).

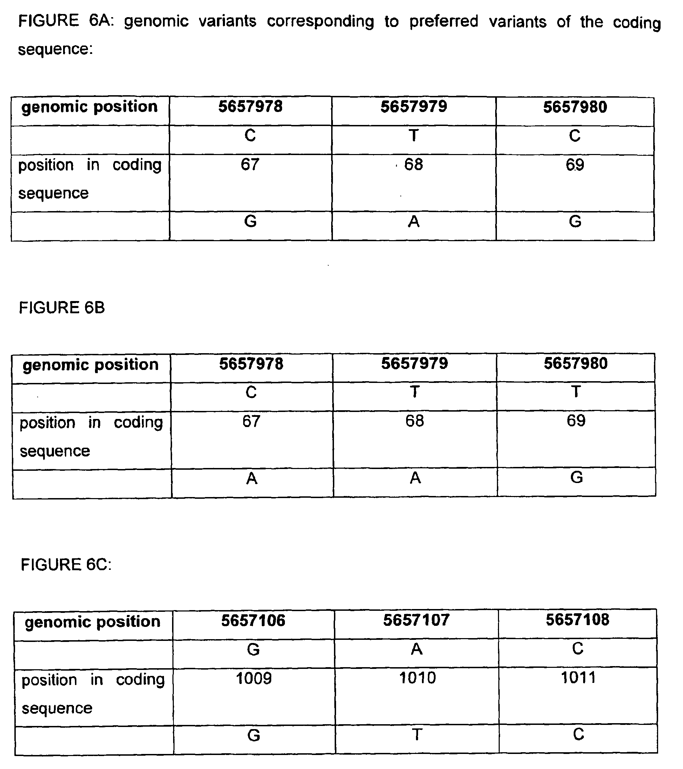

The choice and design of suitable probes is well known in the art (see for example, Sambrook et al., or Current Protocols in Molecular Biology). The same holds true for the preparation of said probes, which are also available from commercial suppliers ( such as MetaBion, Martinsried, Germany). Suitable is e.g. any probe with a higher affinity to a part of the genomic Kir6.2 sequence comprising those nucleotide positions which correspond to positions 67/68/69 and/or 1009/1010/1011 of the coding sequence harbouring at least one nucleotide exchange leading to one of the above amino acid exchanges at position 23 and / or position 337 of the Kir6.2 protein. Suitable probes are for example probably covering the genomic nucleotide Tripletts according to figure 6.

The presence of the amino acid exchange can also be determined by analyzing the RNA of the individual. Suitable techniques are well known in the art and comprise, e.g.: RT PCR techniques, chip hybridization or Northern Blotting.

According to another preferred embodiment of the present invention, the presence of the amino acid exchange is determined by a method comprising or consisting of the following steps:
a. Providing a biological sample containing RNA from an individual.
b. If the RNA is not to be directly analyzed (such as, e.g. by means of in-situ PCR) the RNA is isolated or at least partially purified from the sample according to a).
c. Amplification of a polynucleotide fragment by means of an RT-PCR reaction using primers able to amplify a polynucleotide fragment comprising positions 67 and / or 1009 of the Kir6.2 cDNA sequence
d. Sequencing of the polynucleotide fragment according to c).

The sequencing can be performed according to standard procedures. The visualization of the generated sequence ladder can e.g. be performed by usage of fluorescing or radioactively labeled primers or nucleotides.

The presence or absence of one or more nucleotide exchanges in the Kir6.2 coding sequence leading to an amino acid exchange at position 337 and/or 23 in the Kir6.2 protein can then be determined by analyzing the gained sequence and comparing it to the wild type Kir6.2 sequence.

The choice and design of suitable primers is well known in the art (see for example, Sambrook et al., or Current Protocols in Molecular Biology). The same holds true for the preparation of said primers, which are also available from commercial suppliers ( such as MetaBion, Martinsried, Germany). Suitable is e.g. any primer set able to amplify a polynucleotide comprising at least a part of the Kir6.2 coding sequence spanning positions 67 to 69 and / or positions 1009 to 1011. Preferred primers are for example those according to SEQ ID Nos 9 to 12.

According to another preferred embodiment of the present invention, the presence of the amino acid exchange is determined by a method comprising or consisting of the following steps:
a. Providing a biological sample containing RNA from an individual.
b. Isolating RNA from the sample according to a).
c. Immobilizing the RNA on a carrier.
d. Hybridizing the immobilized RNA to one or more probes able to bind under stringent conditions to Kir6.2 RNA coding for a Kir6.2 polypeptide harbouring another amino acid than Glutaminic Acid at position 23 and/or Valine at position 337 of the with higher affinity than to Kir6.2 RNA coding for a Kir6.2 polypeptide harbouring Glutaminic Acid at position 23 and/or Valine at position 337.

The hybridization can be visualized according to standard procedures such as autoradiography or fluorescence imaging using accordingly labeled probes.

The presence or absence of a nucleotide exchange in the Kir6.2 coding Sequence leading to an amino acid exchange at position 337 and/or 23 in the Kir6.2 protein can then be determined by analysis of the hybridization pattern and / or signal intensity and comparing it to that of a control sample containing Kir6.2 wildtype RNA.
The RNA can for example directly be immobilized on a chip matrix or a suitable membrane before hybridization, or it can be put on a gel matrix and blotted to a suitable membrane after performing gel electrophoresis (Northern Blotting). For reference see e.g. the literature listed below.

The choice and design of suitable probes is well known in the art (see for example, Sambrook et al., or Current Protocols in Molecular Biology). The same holds true for the preparation of said probes, which are also available from commercial suppliers such as MetaBion, Martinsried, Germany. Suitable is e.g. any probe with a higher affinity to a part of the Kir6.2 RNA sequence comprising positions 67 to 69 and/or positions 1009 to 1011 harbouring at least one nucleotide exchange leading to one of the above amino acid exchanges at position 23 and / or position 337 of the Kir6.2 protein.

The presence of the amino acid exchange can also be determined by analyzing the proteome of the individual (i.e. the proteins expressed within the individual). Suitable techniques are well known in the art and comprise, e.g.: Proteomic chip analysis, immunohistological or immunochemical techniques from protein, cell or tissue samples or Western Blot analyses. For reference it is referred, e.g., to the literature listed below.

According to another preferred embodiment of the present invention, the presence of the amino acid exchange is determined by a method comprising or consisting of the following steps:
a. Providing a biological sample containing protein from an individual.
b. Isolating the protein from the sample according to a)
c. Immobilizing it on a carrier.
d. Performing a binding reaction with an antibody able to bind with higher affinity to Kir6.2 harbouring another amino acid than Glutaminic Acid at position 23 and/or than Valine at position 337 of its polypeptide chain than to a Kir6.2 harbouring Glutaminic Acid at position 337 and/or Valine at position 337 of its polypeptide chain (under conditions not allowing for binding to wildtype protein or with much less affinity);
e. Making visible the binding of the antibody to the protein; (e.g. by means of a labeled first antibody or a labeled second antibody, etc.)

The protein can e.g.-directly be immobilized on a chip matrix or a suitable membrane or another type of carrier, such as an ELISA plate. The protein can also be put on a gel, and be transferred to and immobilized on a carrier (such as a suitable membrane) after gel electrophoresis has been performed (Western Blot).

According to yet another preferred embodiment of the present invention, the presence of the amino acid exchange is determined by a method comprising or consisting of the following steps:
a. Providing a histological sample from an individual.
b. Performing a binding reaction with an antibody able to bind with higher affinity to Kir6.2 harbouring another amino acid than Glutaminic Acid at position 23 and/or than Valine at position 337 of its polypeptide chain than to a Kir6.2 harbouring Glutaminic Acid at position 337 and/or Valine at position 337 of its polypeptide chain (under conditions not allowing for binding to the wild type Kir6.2 protein or at least with much less affinity).
c. Making visible the binding of the antibody to the protein; (e.g. by means of a labeled first or secondary antibody).

The presence or absence of the mutation can than be determined by analyzing the labeled histological sample and comparing the intensity of the signal with that of a control sample (e.g. a sample from the same patient treated with a mock first antibody or antiserum (i.e. a first antibody or antiserum that is not reactive for Kir6.2 harboring the mutation, or reacts with Kir6.2 harboring the mutation to a much lesser extent than the specific first antibody or antiserum) or a first antibody specific for the wild type Kir6.2 protein and / or a control sample which is known to contain only the wild type Kir6.2 protein).

The detection of the binding is preferably performed by means of immunohistochemical or immunoradiological methods.

According to a preferred embodiment of the above methods, the amino acid exchange at position 23 is from Glu (wildtype) to Lys; it is also preferred, if the amino acid exchange at position 337 is from Val (wildtype) to Ile.

With respect to the different aspects of present invention, the coronary heart disease is preferably a coronary artery disease, and it is yet more preferred, if it is angina pectoris.

According to further preferred embodiments of the different aspects of the present invention the presence of Kir6.2-23-KK (Kir6.2 protein variants having Lysine (K) at position 23 of the protein as a consequence of a nucleotide polymorphism on both alleles of the Kir6.2 gene) and / or Kir6.2-337-II (Kir6.2 protein variants having Isoleucine (I) at position 337 of the protein as a consequence of a nucleotide polymorphism on both alleles of the Kir6.2 gene) is determined in the sample.

The sample can be any sample containing Kir6.2 harbouring the respective polymorphism(s). It is advantageous, if the samples are prepared in way as to allow for an easy testing for the respective polymorphism(s). Suitable examples depend on the applied detection method and are e.g. histological samples, cell or tissue extracts or cells, preferably if isolated from the human body. Suitable techniques for the identification of the respective polymorphisms, such as PCR, array techniques on nucleic acid or protein basis, immunohistological or immunochemical techniques using suitable antibodies are known to the person of skill in the art. The same holds true for the choice and production of suitable primer sets and antibodies or anti sera. With respect to such techniques it is e.g. referred to the literature listed below.

Another aspect concerns a test kit for testing the presence of another amino acid than Glutaminic Acid at position 23 and / or another amino acid than Valine at position 337 in the Kir6.2 protein, Kir6.2-23-KK or Kir6.2-337-II, wherein the kit contains at least a means for the detection of said presence within the protein. In the context of the present invention, a kit of parts (in short form: kit) is understood to be any combination of the components identified in this application, which are combined, coexisting spatially, to a functional unit, wherein the kit can contain further components.

The means can e.g. be an antibody discriminating in its binding characteristics between Kir6.2 harbouring Glutaminic Acid at position 23 and/or Valine at position 337 and a Kir6.2 protein harbouring another amino acid than Glutaminic Acid at position 23 and/or Valine at position 337. The manufacture of specific antibodies is well known in the art. For reference, it is e.g. referred to the literature listed below. Moreover, it is preferred if the kit contains a labeled secondary antibody. The kit can further contain suitable reagents, buffers, etc. for performing the binding and / or labelling reactions and the like. It is also suitable if instructions for the use (such as a manual or sheet disclosing the preferred reaction conditions, buffer compositions, etc.) are included.

According to a preferred example, the means is an antibody (i.e. a monoclonal antibody, a polyclonal antiserum or a recombinant antibody) differentiating in its binding characteristics between wildtype Kir6.2 and Kir6.2 harbouring an amino acid exchange at position 23 and/or 337. Such an antibody can e.g. bind with higher affinity a Kir6.2 protein that is wild type with respect to position 23 and / or 337 or it can bind with higher affinity to one or more of the less frequent variants with respect to position 23 and / or 337. It also advantageous, if a set of antibodies is included in the test kit, wherein each antibody is able to detect specifically one of the polymorphisms; practically, a control antibody recognizing only the wildtype protein is also included (and possibly also a mock antibody or anti serum for determining background signals), furthermore useful or necessary reagents for performing the detection technique of choice (such as protein blots, array techniques or other immunological or immunohistochemical techniques) can be included, too. The access to or manufacture of suitable antibodies and the choice of useful or necessary reagents for assembling test kits is well known in the art. Moreover the manufacture of antibodies with desired binding characteristics is commercially performed by different suppliers, such as BioTrend, Cologne, Germany.

Another aspect of the invention concerns the use of a test kit for testing the presence of a variation in the Kir6.2 nucleotide sequence on one or both alleles of the Kir6.2 gene leading to a Kir6.2 polypeptide sequence harbouring another amino acid than Glutaminic Acid at position 23 and/or another amino acid than Valine at position 337 in the Kir6.2 protein, wherein the kit contains at least a means for the detection of said variation in the nucleotide sequence.

Another means is e.g. a suitable sequencing primer or a primer set for the specific amplification of DNA or RNA of only wildtype Kir6.2 and/or a primer set for the amplification of DNA or RNA of only Kir6.2 harbouring a mutation at position 23 and/or 337. The choice of and access to suitable primers, able of being used for the specific sequencing or amplification of certain sequences is an ability of the person of skill in the art (see e.g. primers and conditions according to example 2). Useful and necessary reagents for performing the sequencing reaction or the amplification, such as a polymerase, buffers, nucleotides etc. can also be included in the test kit (see e.g. primers and conditions according to example 2). It is very advantageous, if the kit contains an assembly of different sequencing primers or PCR primer sets being specific for different Kir6.2 polymorphisms, such that the presence of different types of polymorphisms within Kir6.2 can be determined.

According to another preferred embodiment, the means is a nucleic acid probe recognizing wildtype Kir6.2 and / or Kir6.2 harbouring a mutation at position 23 and/or 337. The access to and choice of useful probes for performing the detection technique of choice (e.g. all kinds of nucleic acid blots, array techniques or other kind of chip techniques) lies within the skills of the person of the art, too. So does the choice of suitable reagents, which can be included in the test kit as well. Preferably, a set of different probes recognizing different polymorphisms is included in the test kit.

The amino acid at position 23 is preferably Lys and that at position 337 is preferably Ile.

It is preferred if the nucleotide sequence of Kir6.2 carries a G at position 67 of the coding sequence. It is also preferred, if the nucleotide sequence of Kir6.2 carries an A at position 1009 of the coding sequence.

According to a preferred embodiment, the test kit contains a primer set able to amplify a polynucleotide fragment comprising positions 67 and / or 1009 of the Kir6.2 coding sequence. Preferred primers are e.g. those according to SEQ ID Nos. 9 to 12.

According to another preferred embodiment, the test kit contains at least one primer set able to amplify a polynucleotide fragment comprising positions 5657106/7/8 and/ or 5657978/79/80 of the genomic Kir6.2 sequence according to SEQ ID No. 13. Preferred primers are e.g. those according to SEQ ID Nos. 9 to 12.

According to another preferred embodiment, the test kit contains one or more nucleic acid probes specifically recognizing Kir6.2 genomic DNA or RNA with a nucleotide sequence coding for another amino acid than Glutaminic Acid at position 23 and/or Valine at position 337 under stringent conditions.

Another aspect concerns an isolated Kir6.2 polypeptide or a fragment thereof carrying Lysine at position 23 and Isoleucine at position 337 of the polypeptide sequence. A preferred embodiment of the invention concerns an isolated Kir6.2 polypeptide according to SEQ ID No.7 or a fragment thereof comprising amino acids positions 23 and / or 337.

Yet another aspect refers to an isolated polynucleotide coding for one of the above K23/I337- Kir6.2 polypeptides or fragments thereof comprising positions 23 and 337. A preferred embodiment of the invention concerns an isolated Kir6.2 polynucleotide according to SEQ ID No.8 or a fragment thereof comprising the nucleotide positions 67 and 1009 (preferably also positions 68, 69, 1010 and 1011), or an isolated polynucleotide hybridizing to the above DNA sequences or their complementary strands under conditions of high stringency.

Another aspect concerns an isolated Kir6.2 polypeptide or a fragment thereof carrying Glutaminic Acid at position 23 and Isoleucine at position 337 of the polypeptide sequence. A preferred embodiment of the invention concerns an isolated Kir6.2 polypeptide according to SEQ ID No.5 or a fragment thereof comprising amino acids positions 23 and / or 337.

Yet another aspect refers to an isolated polynucleotide coding for one of the above E23/I337- Kir6.2 polypeptides or fragments thereof comprising positions 23 and 337. A preferred embodiment of the invention concerns an isolated Kir6.2 polynucleotide according to SEQ ID No.6 or a fragment thereof comprising the nucleotide positions 67 and 1009 (preferably also positions 68, 69, 1010 and 1011), or an isolated polynucleotide hybridizing to the above DNA sequences or their complementary strands under conditions of high stringency.

Another aspect concerns a probe for the detection of nucleotide variations within the Kir6.2 gene or RNA comprising or consisting of at least 17, preferably 19 to 100 consecutive nucleotides of the Kir6.2 sequence spanning position 67 and / or 1009 of the Kir6.2coding sequence or spanning positions 5657106/07/08 and/or 5657978/79/80 of the genomic Kir6.2 sequence.

Another aspect concerns primers or primer sets for the amplification of Kir6.2 polynucleotides wherein the amplified polynucleotides comprise at least position 67 and / or 1009 of the coding Kir6.2 sequence and/or position 5657106/07/08 and/or 5657978/79/80 of the genomic Kir6.2 sequence.

In the following, the present invention is explained in more detail by means of several examples, which are not meant to limit the scope of the present invention.

### Examples:

The Kir6.2 polymorphisms at position 23 and at position 337 of the Kir6.2 protein (NCBI accession number for wild type protein sequence: D 50582 (SEQ. ID No 1.; Figure 1A); NCBI accession number for coding sequence: D 05852 (SEQ. ID No. 2; Figure 1B)) were analyzed in a patient cohort with type I and II diabetes (mostly type II, i.e. diabetes mellitus).

### Example 1: Study subjects (study population)

The genomic DNA of 335 patients was screened for SNPs (single nucleotide polymorphism) in the Kir6.2 gene leading to the protein variants Kir6.2-E23K and Kir6.2-V337I. Inclusion criteria have been: Caucasian individual of German ancestry, stable clinical condition and coronary angiogram. Exclusion criteria have been: acute illness other than ACS, chronic non-cardiac disease (i.e. rheumatic arthritis) and history of malignant disease within the previous five years. Basic characteristics of this patient cohort are outlined in Table 2. All patients signed written informed consent.

### Example 2: SNP detection by sequencing and analysis

### 2.1: Amplification of genomic region with polymorphism of interest

Amplification primers:
A: For the detection of a nucleotide exchange at positions 5657978/79/80 of the Kir6.2 gene sequence with the accession number NT_009307.
   Forward primer M67: 5'-AGGTGGAGGTAAGGAAGAG-3' (SEQ ID. No. 9)
   Reverse primer M67: 5'-GGTGAAGATGAGCAATGTG-3' (SEQ ID. No. 10)
B: For the detection of nucleotide exchange at positions 5657106/07/08 of the Kir6.2 gene sequence with the accession number NT_009307.
   Forward primer M1009: 5'-GGGTGGCAACAGCATCTTC-3' (SEQ ID. No. 11)
   Reverse primer M1009: 5'-TGGCTCAGGACAGGGAATC-3' (SEQ ID. No.12)

The primers can also be applied for amplification of Kir6.2 coding sequences.

### PCR protocol for amplification:

All reagents are from Applied Biosystems (Foster City, USA): 20 ng of genomic DNA; 1 unit of TaqGold polymerase; 1 x Taq polymerase buffer; 500 µM of dNTP; 2,5 mM of MgCI2; 200 nM of each amplification primer pair (for sequence see Amplification primer pairs 1.A and 1.B above); H₂O ad 5 µl.

Amplification program for PCR/genotyping:

| | |
|---|---|
| 95°C x 10min | x 1 cycle |
| | |
| 95°C x 30sec | |
| 70°C x 30sec | x 2 cycles; |
| | |
| 95°C x 30sec | |
| 65°C x 30sec | x 2 cycles; |
| | |
| 95°C x 30sec | |
| 60°C x 30sec | x 2 cycles; |
| | |
| 95°C x 30sec | |
| 56°C x 30sec | |
| 72°C x 30sec | x 40 cycles; |
| | |
| 72°C x 10min | |
| 4°C x 30sec | x 1 cycle; |

### 2.2: Identification of polymorphisms of interest

### Protocol for minisequencing and detection of polymorphisms:

All reagents are from Applied Biosystems (Foster City, USA). 2 µl of purified PCR product; 1,5 µl BigDye terminator kit; 200 nM of one sequencing primer (for sequence see forward or reverse amplification primer 1.A and 1.B above); H₂O ad 10 µl.

### Amplification program for sequencing:

96°C x 2min x 1 cycle;
96°C x 10sec
   55°C x 10sec
   65°C x 4min x 30 cycles;
72°C x 7min
   4°C x 30sec x 1 cycle;

### Analysis of sequencing products:

Sequences were analyzed first with sequencing analysis software (Applied Biosystems, Foster City, USA) for raw data extraction and processed with Phred (base caller), Phrap (assembler), Polyphred (SNP caller) and Consed (results viewer). Phred, Phrap, Polyphred and Consed are software designed at WashU by Phil Green (http://www.genome.washington.edu).
The PCR reactions led to the identification of an exchange from G to A at position 67 and from A to G at position 1009 of the coding sequence.

### 2.3: Correlation of amino acid exchanges within the Kir6.2 Protein with coronary heart disease

In order to analyze the influence of Kir6.2 polymorphisms on clinical outcome in patients with cardiovascular and metabolic disorders, the inventors performed correlation analyses of Kir6.2 polymorphisms E23K, L270V and I337V in a patient cohort of 335 individuals with type I or II diabetes. Out of more than 350 clinical parameters, angina pectoris correlated significantly with patients homozygous for Lysine (K) at position 23 and/or Valine (V) at position 337 of the Kir6.2 protein.

Kir6.2-23-EE (wild type for position 23) defines the group of individuals, in which both of the Kir6.2 alleles code for a Kir6.2 gene variant leading to Glutamic Acid (Glu or E) at position 23 of the Kir6.2 protein, this group is homozygous for this Kir6.2 polymorphism at position 23 of the Kir6.2 protein.

Kir6.2-23-EK defines the group of individuals, in which one of the Kir6.2 alleles codes for a Kir6.2 gene variant leading to Glutamic Acid (Glu or E) at position 23 of the Kir6.2 protein and the other Kir6.2 allele codes for a Kir6.2 gene variant leading to Lysine (Lys or K) at position 23 of the Kir6.2 protein, this group is heterozygous for these Kir6.2 polymorphisms at position 23 of the Kir6.2 protein.

Kir6.2-23-KK defines the group of individuals, in which both of the Kir6.2 alleles code for a Kir6.2 gene variant leading to Lysine (Lys or K) at position 23 of the Kir6.2 protein, this group is homozygous for this Kir6.2 polymorphism at position 23 of the Kir6.2 protein. Kir6.2-23-KK defines also a biological sample of patients in which both of the Kir6.2 alleles code for a Kir6.2 gene variant leading to Lysine (Lys or K) at position 23 of the Kir6.2 protein.

Kir6.2-337-II defines the group of individuals, in which both of the Kir6.2 alleles code for a Kir6.2 gene variant leading to Isoleucine (Ile or I) at position 337 of the Kir6.2 protein, this group is homozygous for this Kir6.2 polymorphism at position 337 of the Kir6.2 protein. Kir6.2-337-II defines as well a biological sample of patients in which both of the Kir6.2 alleles code for a Kir6.2 gene variant leading to lsoleucine (Ile or I) at position 337 of the Kir6.2 protein.

Kir6.2-337-IV defines the group of individuals, in which one of the Kir6.2 alleles codes for a Kir6.2 gene variant to Isoleucine (Ile or I) at position 337 of the Kir6.2 protein and the other Kir6.2 allele codes for a Kir6.2 gene variant leading to Valine (Val or V) at position 337 of the Kir6.2 protein, this group is heterozygous for these Kir6.2 polymorphisms at position 337 of the Kir6.2 protein.

Kir6.2-337-W (wild type for position 337) defines the group of individuals, in which both of the Kir6.2 alleles code for a Kir6.2 gene variant leading to Valine (Val or V) at position 337 of the Kir6.2 protein, this group is homozygous for this Kir6.2 polymorphism at position 337 of the Kir6.2 protein.

### Statistical approaches for genotype/phenotype correlation:

All analyses were done with SAS statistical package (Version 6.12, SAS Institute GmbH, Heidelberg/Germany). For the detection of associations between genetic polymorphisms and a large number of clinical relevant parameters, descriptive statistics were computed (median, quartiles) and Wilcoxon-rank-sum-tests were performed. Wilcoxon-rank-sum-test is used for the comparison of two independent samples. The computation of the test statistic is based on ranks in the pooled sample. The search for associations between the SNPs and risk factors and diseases was done in a similar way. The Chi-Square-Test was performed and numbers and percentages were calculated to describe the data. The Chi-Square-Test is a statistical test for calculating the dependence of two variables. The values of the variables are contained in two or more classes. To analyze the association of those variables, a contingency table is used. This table contains as many rows as the number of realizations of the first variable and as many columns as the number of realizations of the second variable. Every cell contains a special patient's characteristic. To construct a test statistic, the differences of calculated and observed frequencies are computed. After inspecting the results, relevant variables were selected. To take account of confounding co-variables, logistic regression was used to validate the results. The logistic regression method is used to analyze the influence of several explanatory variables on a certain response variable. The associated statistical test gives a p-value. The interpretation of this p-value is that there is a significant influence of the associated explanatory variable.

For a binary variable, the odds ratio has been calculated. The odds ratio is the ratio of the odds that an event will occur in one group to the odds that the event will occur in the other group.

### Example 3: Analyses

The distribution of Kir6.2 variants E23K and V3371 in 335 individuals is shown in Table 2. As outlined in Tables 3 to 8, a strong linkage of Kir6.2-23-KK and Kir6.2-337-II, Kir6.2-23-KK and Kir6.2-337-VI and Kir6.2-23-EE and Kir6.2-337-VV can bee seen. Therefore, all statistical analysis data obtained for e.g. Kir6.2-23-KK must be similar to those of Kir6.2-337-II.

Diabetes patients carrying Kir6.2-23-KK and Kir5.2-337-II show an increased association with angina pectoris. Statistical significance calculated with Chi-square test for the association of Kir6.2-23-KK genotype in diabetes patients with angina pectoris is p-value = 0.015 and for the association of Kir6.2-337-II genotype in diabetes patients with angina pectoris is p-value = 0.012 (Table 3 and Table 6).

Logistic regression for analyzing the influence of confounding factors, such as myocardial infarction and hypertension resulted in a p-value = 0.0088 for the association of Kir6.2-23-KK genotype in diabetes patients with angina pectoris and p-value = 0.0072 for the association of Kir6.2-337-II genotype in diabetes with angina pectoris (Table 4 and Table 7).

The odds ratios of an increased risk for angina pectoris in diabetic patients with Kir6.2-23-KK genotype is 1.601 and with Kir6.2-337-II genotype 1.615 (Table 5 and Table 8).

The data shown here demonstrate that mutations within the Kir6.2 gene leading from an exchange at position 23 from Glutamine to Lysine, especially Kir6.2-23-KK and mutations with the Kir6.2 gene leading to an amino acid exchange at position 337 from Valine to Isoleucine, especially Kir6.2-337-II, represent risk markers for angina pectoris in diabetic patients. Since angina pectoris is a sign for coronary heart disease, it can be expected that these markers are indicative of coronary heart disease in general. Moreover it can be expected, that other mutations within the Kir6.2 gene leading to an amino acid exchange at position 23 within the Kir6.2 protein, especially those exchanging the acidic Glutamine for a nonpolar or preferably a basic amino acid have the same or a similar effect. The same can be expected for amino acid exchanges at position 337 exchanging the Valine for other, especially acidic or basic or more bulky aliphatic amino acids. It is very likely that these amino acid exchanges correlate very closely with the onset of coronary heart disease, especially angina pectoris, in non-diabetic patients, too.

Literature:
Yamada Y, Kuroe A, Li Q, Someya Y, Kubota A, Ihara Y, Tsuura Y, Seino Y (2001). Genomic variation in pancreatic ion channel genes in Japanese type 2 diabetic patients. Diabetes Metab Res Rev 17:213-216.

Hani EH, Boutin P, Durand E, Inoue H, Permutt MA, Velho G, Froguel P (1998). Missense mutations in the pancreatic islet beta cell inwardly rectifying K+ channel gene (KIR6.2/BIR): a meta-analysis suggests a role in the polygenic basis of Type II diabetes mellitus in Caucasians. Diabetologia 41:1511-1515.

### Standard literature for laboratory methods

If not indicated otherwise, standard laboratory methods were performed according to the following standard literature:
Sambrook et al. (1989) Molecular Cloning: A Laboratory Manual. Second edition. Cold Spring Harbor Laboratory Press, Cold Spring Harbor, NY. 545 pp;
Current Protocols in Molecular Biology; regularly updated, e.g. Volume 2000; Wiley & Sons, Inc; Editors: Fred M. Ausubel, Roger Brent, Robert Eg. Kingston, David D. Moore, J.G. Seidman, John A. Smith, Kevin Struhl.

Current Protocols in Human Genetics; regularly uptdated; Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith.

Current Protocols in Protein Science; regularly updated; Wiley & Sons, Inc; Editors: John E. Coligan, Ben M. Dunn, Hidde L. Ploegh, David W. Speicher, Paul T. Wingfield. Molecular Biology of the Cell; third edition; Alberts, B., Bray, D., Lewis, J., Raff, M., Roberts, K., Watson, J.D.; Garland Publishing, Inc. New York & London, 1994;

Short Protocols in Molecular Biology, 5th edition, by Frederick M. Ansubel (Editor), Roger Brent (Editor), Robert E. Kingston (Editor), David D. Moore (Editor), J.G. Seidman (Editor), John A. Smith (Editor), Kevin Struhl (Editor), October 2002, John Wiley & Sons, Inc., New York"

### Figure Legends:

### Figure 1:

Protein sequence and coding nucleotide sequence of Kir6.2 (KCNJ11) harbouring the most frequent variants at position 23 (Glutamic Acid) and position 337 (Valine) of the polypeptide chain (corresponding to the codons GAG at positions 67/68/69 and GTC at positions 1009/1010/1011 of the coding sequence). These variants are referred to as Kir6.2-23K-337V (or KK and/or VV if both alleles are alike with respect to position 23 and/or 337). The protein accession number (NCBI protein database) of this Kir6.2 variant is D50582. Positions 23 and 337 of the polypeptide sequence and corresponding nucleotide positions, as well as the ATG within the coding sequence are underlined.

### Figure 2:

Protein sequence and coding nucleotide sequence of Kir6.2 (KCNJ11) harbouring the variant Lysine at position 23 and the most frequent variant Valine at position 337 (corresponding to the codons AAG at positions 67/68/69 and GTC at positions 1009/1010/1011 of the coding sequence). The protein sequence accession number (NCBI protein database) of this Kir6.2 variant is XP_006398 (Figure 2A; SEQ ID No.2). Accession number for the nucleotide sequence (NCBI nucleotide database) is XM_006398 (Figure 2B; SEQ ID No.4). Positions 23 and 337 of the polypeptide sequence and corresponding nucleotide positions within the coding sequence are underlined.

### Figure 3:

Protein sequence and coding nucleotide sequence of Kir6.2 (KCNJ11) harbouring the most frequent variant Glutamic Acid at position 23 and the variant Isoleucine at position 337 (corresponding to the codons GAG at positions 67/68/69 and ATC at positions 1009/1010/1011 of the coding sequence). The protein sequence corresponds to that of the "wild type" Kir6.2 according to sequence accession number D50582 except for position 337, which carries an Isoleucine instead of a Valine. The coding sequence corresponds to that of the "wild type" Kir6.2 according to sequence accession number D50582 except for position 1009, which carries an Adenosine instead of a Guanosine. Positions 23 and 337 of the polypeptide sequence and corresponding nucleotide positions within the coding sequence are underlined.

### Figure 4:

Protein sequence and coding nucleotide sequence of Kir6.2 (KCNJ11) harbouring the variant Lysine at position 23 and the variant Isoleucine at position 337 (corresponding to the codons AAG at positions 67/68/69 and ATC at positions 1009/1010/1011 of the coding sequence). The protein sequence corresponds to that of the "wild type" Kir6.2 according to sequence accession number D50582 except for position 23, which carries a Lysine instead of a Glutaminic Acid and position 337, which carries an Isoleucine instead of a Valine. The coding sequence corresponds to that of the "wild type" Kir6.2 according to sequence accession number D50582 except for position 67, which carries an Adenosine instead of a Guanosine and position 1009, which carries an Adenosine instead of a Guanosine. Positions 23 and 337 of the polypeptide sequence and corresponding nucleotide positions within the coding sequence are underlined.

### Figure 5:

DNA primers M67 forward (SEQ ID No.9) and reverse (SEQ ID No.10) for the detection of nucleotide exchanges at those positions of the Kir6.2 genomic sequence that correspond to positions 67 / 68/ 69 of the Kir6.2 coding sequence and DNA primers M1009 forward (SEQ ID No.11) and reverse (SEQ ID No.12) for the detection of nucleotide exchanges at those positions of the Kir6.2 genomic sequence that correspond to positions 1009/1010/1011 of the Kir6.2 coding sequence according to sequence accession number. With respect to SEQ ID No. 25, the positions are 5657106/107/108 and 5657978/79/80, respectively. The Primer set are also applicable for the specific amplification of Kir6.2 cDNA according to NCBI sequence accession number D50582. Primers M67 forward (SEQ ID No. 9) and reverse (SEQ ID No. 10) amplify a region comprising nucleotides 67/68/69 of the Kir6.2 cDNA for the detection of nucleotide exchanges within the Kir6.2 gene on RNA level. Primers M1009 forward (SEQ ID No. 11) and reverse (SEQ ID No. 12) amplify a region comprising nucleotides 1009/1010/1011 of the Kir6.2 cDNA for the detection of nucleotide exchanges within the Kir6.2 gene on RNA level.

### Figure 6:

Overview of corresponding nucleotide positions of genomic and coding sequences of some Kir6.2 variants. The numbering of the positions within the genomic sequence has been derived from the Homo sapiens chromosome 11 genomic contig under NCBI accession number NT_009307 (see also figure 10 for the part of the contig. comprising the Kir6.2 genomic locus).

### Figure 7:

Variations of the kKir6.2 nucleotide sequence leading to an amino acid exchange from Glutamic Acid to Lysine at position 23 and from Valine to Isoleucine at position 337 of the polypeptide chain. Nucleotide positions refer to the Kir6.2 coding sequence. These positions correlate with positions 5657106/7/8 and 5657978/79/80 of the genomic Kir6.2 sequence according to SEQ ID No. 25 (wherein SEQ ID No. 25 codes for valine at pos. 337 of the protein sequence). Most frequently occurring variants (i.e. the wild type) are written in normal letters, deviations from this wild type in the nucleotide or amino acid sequence are given in bold letters. With respect to amino acid 23, the single nucleotide exchange G67A results in a Lysine being incorporated in the polypeptide chain, whereas the exchange G69A alone does not result in a change of the amino acid sequence. This type of mutation is commonly referred to as silent mutation. If, on the other hand, both of the above nucleotide exchanges occur together, a Lysine is incorporated at position 23 as well. With respect to position 337 of the amino acid chain, the single nucleotide exchanges C1011T, C1011A and C1011G are silent, whereas the single nucleotide exchange G1009A leads to the incorporation of an Isoleucine at position 337 of the polypeptide chain, even if exchanges C1011T or C1011A occur at together with the G1009A exchange. Other exchanges at positions 67/68/69 and / or 1009/1010/1011 are possible, resulting in the incorporation of other amino acids into the polypeptide chain than G or K at position 23 or V or I at position 337, respectively (these correlations resulting from the genetic code are commonly known in the state of the art).

### Figure 8:

Genomic sequence of Kir6.2 (KCNJ11) locus harbouring the nucleotides GAC at positions 5657106/107/108 (corresponding to the codon GTC at positions 1009/1010/1011 of the coding sequence and thus coding for a protein variant harbouring the amino acid Valine at position 337 of the polypeptide sequence) and harbouring the nucleotides CTT at positions 5657978/79/80 (corresponding to the codon AAG at positions 67/68/69 of the coding sequence and thus coding for a protein variant harbouring the amino acid Lysine at position 23 of the peptide sequence). The indicated nucleotide triplets are typed bold and underlined. The hybridization positions of the PCR primers according to SEQ ID Nos. 9 to 12 are typed bold and underlined, as well. The primer sequences are chosen as to allow for the amplification of polynucleotides using as template the Kir6.2 cDNA, as well. The sequence accession number (NCBI nucleotide database) making publicly available homo sapiens chromosome 11 genomic contiguous Sequence comprising this Kir6.2 genomic variant is NT _009307 (SEQ ID No.25).

### Legend to the tables:

### Table 1:

Basic characteristics of the patient cohort for which all analyses have been performed.

### Table 2:

Distribution of Kir6.2 variants in the analyzed diabetes patient cohort.

### Table 3:

Association of Kir6.2 variants at position 23 of the protein with angina pectoris in diabetes patients calculated by Chi-square test. An increased frequency of Kir6.2-23-KK carriers in the angina pectoris positive group could be observed.

### Table 4:

Calculation of statistical significance for Kir6.2-23-KK association with angina pectoris in diabetes patients by logistic regression.

### Table 5:

Calculation of odds ratios for the risk of diabetes patients having angina pectoris of Kir6.2-23-KK carriers compared to Kir6.2-23-EK and Kir6.2-23-EE carriers.

### Table 6:

Association of Kir6.2 variants at position 337 of the protein with angina pectoris in diabetes patients calculated by Chi-square test. An increased frequency of Kir6.2-337-II carriers in the angina pectoris positive group could be observed.

### Table 7:

Calculation of statistical significance for Kir6.2-337-II association with angina pectoris in diabetes patients by logistic regression.

### Table 8:

Calculation of odds ratios for the risk of diabetes patients having angina pectoris of Kir6.2-337-II carriers compared to Kir6.2-337-VI and Kir6.2-337-VV carriers.

**Table 1**

| | | n | % |
|---|---|---|---|
| Total | | 335 | |
| Gender | Female | 100 | 29.9 |
| | Male | 235 | 70.1 |
| Age* | | 66.3 (39.5-87.2) | |
| BMI* (Body Mass Index) | | 29.2 (17.5-57.1) | |
| Hypertension | | 219 | 65.4 |
| Smoker | | 224 | 66.9 |
| Angina pectoris | | 210 | 62.7 |
| Diabetes Type I or II | | 335 | 100 |
| Stable coronary artery disease (CAD) | CCS 1 | 126 | 37.6 |
| | CCS 2 | 127 | 37.9 |
| | CCS 3 | 62 | 18.5 |
| | CCS 4 | 20 | 6.0 |
| Acute coronary syndrome (ACS) | no ACS (no CAD/stable CAD/MI>15d) | 208 | 62.1 |
| | tropT-UA (no acute MI) | 77 | 23.0 |
| | tropT+UA (no clinical MI) | 21 | 6.3 |
| | post acute MI (1-15 d) | 29 | 8.7 |

| | | | |
|---|---|---|---|
| *Median and Quartiles (Q1-Q3) | | | |

**Table 2:**

| | **Kir6.2-337-VV** | **Kir6.2-337-VI** | **Kir6.2-337-II** |
|---|---|---|---|
| **Kir6.2-23-EE** | 138 | 0 | 0 |
| **Kir6.2-23-EK** | 2 | 148 | 1 |
| **Kir6.2-23-KK** | 0 | 0 | 46 |

**Table 3:**

| | **Angina pectoris (AP)** | | |
|---|---|---|---|
| | Number of patients without AP (%) | Number of patients with AP (%) | p-value |
| **Kir6.2-23-EE** | 63 (50.4) | 75 (35.7) | **0.015** |
| **Kir6.2-23-EK** | 51 (40.8) | 100 (47.6) | |
| **Kir6.2-23-KK** | 11 (8.8) | 35 (16.7) | |

**Table 4:**

| | **p-value (logistic regression)** |
|---|---|
| **Kir6.2-23-KK** | **0.0088** |
| Male gender | 0.1291 |
| Smoker | 0.0914 |
| Arterial hypertension | 0.2098 |
| Myocardial Infarction | 0.1988 |
| ACE inhibitor | 0.9148 |
| Beta-Blocker | 0.4437 |
| Tot. Cholesterol >=240 or drug history | 0.0507 |

**Table 5:**

| | odds ratio | 95%-confidence interval | | |
|---|---|---|---|---|
| | | lower | Upper | p-value |
| **Angina pectoris** | 1.601 | 1.126 | 2.277 | **0.0088** |

**Table 6:**

| | **Angina pectoris (AP)** | | |
|---|---|---|---|
| | Number of patients without AP (%) | Number of patients with AP (%) | p-value |
| **Kir6.2-337-VV** | 64 (51.2) | 76 (36.2) | **0.012** |
| **Kir6.2-337-VI** | 50 (40.0) | 98 (46.7) | |
| **Kir6.2-337-II** | 11 (8.8) | 36 (17.1) | |

**Table 7:**

| | **p-value (logistic regression)** |
|---|---|
| **Kir6.2-337-II** | **0.0072** |
| Male gender | 0.1256 |
| Smoker | 0.0902 |
| Arterial hypertension | 0.2211 |
| Myocardial Infarction | 0.2165 |
| Betablocker | 0.4552 |
| Tot. Cholesterol >=240 or drug history | 0.0526 |
| ACE Inhibitor | 0.9250 |

**Table 8:**

| | odds ratio | 95%-confidence interval | | |
|---|---|---|---|---|
| | | lower | Upper | p-value |
| **Angina pectoris** | 1.615 | 1.139 | 2.291 | **0.0072** |

## Claims

1. A method of identifying an increase in risk for angina pectoris in a diabetic patient, wherein the presence of another amino acid than Glutaminic Acid at position 23 and/ort he presence of another amino acid than Valine at position 337 in the Kir6.2 protein is determined in a sample.

2. A method of identifying an increase in risk for angina pectoris in a diabetic patient, wherein the presence of a variation in the Kir6.2 nucleotide sequence on one or both alleles of the Kir6.2 gene leading to a Kir6.2 polypeptide sequence harbouring another amino acid than Glutaminic Acid at position 23 and/or another amino acid than Valine at position 337 in the Kir6.2 protein is determined in a sample.

3. A method of adapting the dosage of a pharmaceutical useful for treating and/or preventing angina pectoris in a diabetic patient, wherein:
I. the presence of a Kir6.2 with.
a)an amino acid other than Glutaminic Acid at position 23 and/or an amino acid other than Valine at position 337 in the Kir6.2 protein,
b)a variation in the nucleotide sequence on one or both alleles of the Kir6.2 gene leading to amino other than Glutaminic Acid at position 23 and/or an amino acid other than Valine at position 337 in the Kir6.2 protein or
c) Kir6.2-23-KK and/or Kir6.2-337-II is determined in a sample of a diabetic patient.

4. The method or use according to one of the claims 1 to 3, wherein the amino at position 23 with respect to SEQ ID No 1 of the Kir6.2 protein is Lys.

5. The method or use according to one of the claims 1 to 4, wherein the amino acid at position 337 with respect to SEQ ID No 1 of the Kir6.2 protein is Ile.

6. The method or use according to one of the claims 2 to 5, wherein the nucleotide sequence carries an A at position 67 with respect to SEQ ID No 2 of the Kir6.2 coding sequence.

7. The method or use according to one of the claims 2 to 6, wherein the nucleotide sequence carries a G at position 1009 with respect to SEQ ID No 2 of the Kir6.2 coding sequence.

8. The method or use according to one of the preceding claims 1 to 7, wherein the diabetes is diabetes mellitus.

9. The method according to one of the preceding claims 1 to 8, wherein the presence of Kir6.2-23-KK and/or Kir6.2-337-11 is determined in the sample.

10. The method according to one of the preceding claims 1 to 9, wherein the sample is a histological sample, a cell or tissue extract or a cell.

11. The method according to one of the claims 1 to 10, wherein the sample is of human origin.

12. The method according to one of the claims 1 to 11, wherein the presence of the amino acid exchange is determined by
a)Amplification of a polynucleotide fragment from a biological sample containing genomic DNA, wherein the chromosomal DNA has preferably been isolated from the sample, by means of a PCR reaction using primers able to amplify a polynucleotide fragment comprising those nucleotide positions of the genomic Kir6.2 sequence that correspond to positions 67/68/69 and/or 1009/1010/1011 with respect to SEQ ID No. 2 of the coding sequence;
b)Sequencing of the polynucleotide fragment according to a);

13. The method according to claim 12, wherein the amplification of the polynucleotide fragment is performed using at least on and preferably two of the primers according to SEQ. ID No. 9 to 12.

14. The method according to claim 12 or 13, wherein the sequencing reaction is performed using one of the primers according to SEQ ID No. 9 to 12.

15. The method according to one of the claims 1 to 11, wherein the presence of the amino acid exchange is determined by
a)Isolating genomic DNA from a biological sample containing genomic DNA
b) Immobilizing it on a carrier
c) Hybridizing to the immobilized DNA one or more probes able to bind to the Kir6.2 Sequence harbouring one or more nucleotide exchanges leading to one or both of said amino acid exchanges with higher affinity than to the wild type Kir6.2 Sequence under stringent conditions.

16. The method according to claim 15, wherein the isolated genomic DNA is
a)Immobilized on a chip matrix, a suitable membrane or
b)Transferred to a gel matrix and blotted to a suitable membrane.

17. The method according to one of the claims 1 to 11, wherein the amino acid exchange is determined by
a)Amplification of a polynucleotide fragment by means of an RT-PCR reaction using primers able to amplify a polynucleotide fragment comprising positions 67 with respect to SEQ ID No. 2 and/or 1009 with respect to SEQ ID No. 2 of the Kir6.2 cDNA sequence and using a biological sample containing RNA from an individual and preferably using isolated RNA from the sample
b)Sequencing of the polynucleotide fragment according to a).

18. The method according to claim 17, wherein at least one of the primers according to SEQ ID No. 9 to 12 is used.

19. The method according to one of the claims 1 to 11, wherein the amino acid exchange is determined by
a) Isolating RNA from a biological sample containing RNA from an individual
b) Immobilising it on a carrier;
c) Hybridising the immobilized RNA to one or more probes able to bind under stringent conditions to Kir6.2 RNA coding for a Kir6.2 polypeptide harbouring another amino acid the Glutaminic Acid at position 23 with respect to SEQ ID No. 1 and/or Valine at position 337 with respect to SEQ ID No. 1 of the Kir6.2 protein with higher affinity than to Kir6.2 RNA coding for a Kir6.2 polypeptide harbouring Glutaminic Acid at position 23 with respect to SEQ ID No. 1 and/or Valine at position 337 with respect to SEQ ID No. 1.

20. The method according to claim 19, wherein the isolated RNA is
a) Immobilized on a chip matrix or a suitable membrane, or
b)Transferred to a gel matrix and afterwards blotted to a suitable membrane.

21. The method according to one of the claims 1 to 11, wherein the amino acid exchange is determined by
a) Isolating protein from a biological sample containing proteins from an individual;
b) Immobilizing it on a carrier;
c) Performing a binding reaction reaction with an antibody able to bind with higher affinity to Kir6.2 harbouring another amino acid than Glutaminic Acid at position 23 with respect to SEQ ID No. 1 and/or than Valine at position 337 with respect to SEQ ID No. 1 of its polypeptide chain than to a Kir6.2 harbouring Glutamanic Acid at position 337 with respect to SEQ ID No. 1 and/or Valine at position 337 with respect to SEQ ID No. 1 of its polypeptide chain;
d) Making visible the binding of the antibody to the protein;

22. The method according to claim 21 wherein the isolated protein is
a) Immobilized on a chip matrix or a suitable membrane;
b)Transferred to a gel matrix and afterwards immobilized on a suitable membrane, or
c) Immobilized on an ELISA plate.

23. The method according to one of the claims 1 to 11, wherein the amino acid exchange is determined by
a) Performing on a histological sample from an individual a binding reaction with an antibody able to bind with higher affinity to Kir6.2 harbouring another amino acid than Glutamanic Acid at position 23 with respect to SEQ ID No. 1 and/or Valine at position 337 with respect to SEQ ID No. 1 of its polypeptide chain than to a Kir6.2 harbouring Glutamanic Acid at position 337 with respect to SEQ ID No. 1 and/or Valine at position 337 with respect to SEQ ID No. 1 of its polypeptide chain;
b) Making visible the binding of the antibody to the protein.

24. The method according to claim 23, wherein the detection of the binding is performed by means of an immunohistochemical or immunoradiological reaction.

25. Use of a test kit for testing the presence of another amino acid than Glutaminic Acid at position 23 with respect to SEQ ID No. 1 and/or another Amino Acid than Valine at position 337 with respect to SEQ ID No. 1 in the Kir6.2 protein, Kir6.2-23-KK or Kir6.2-337-II, wherein the kit contains as least a means for the detection of said presence within the protein, in order to identify an increase in risk for angina pectoris in a diabetic patient.

26. Use of a test kit for testing the presence of a variation in the Kir6.2 nucleotide sequence on one or both alleles of the Kri6.2 gene leading to a Kir6.2 polypeptide sequence harbouring another amino acid than Glutamanic Acid at position 23 with respect to SEQ ID No. 1 and/or another amino acid than Valine at position 337 with respect to SEQ ID No. 1 in the Kir6.2 protein, wherein the kit contains at least a means for the detection of said variation in the nucleotide sequence, in order to identify an increase in risk for angina pectoris in a diabetic patient.

27. Use according to one of the claims 25 or 26 wherein the amino acid at position 23 with respect to SEQ ID No. 1 is Lys.

28. Use according to one of the claims 25 to 27, wherein the amino acid at position 337 with respect to SEQ ID No. 1 is Ile.

29. Use according to one of the claims 25, 27 or 28, wherein the test kit contains an antibody discriminating in its binding characteristics between Kir6.2 harbouring Glutamanic Acid at position 23 with respect to SEQ ID No. 1 and/or Valine at position 337 with respect to SEQ ID No. 1.

30. Use according to one of the claims 26 to 28, wherein the nucleotide sequence of Kir6.2carries G at position 67 with respect to SEQ ID No. 2 of the coding sequence.

31. Use according to one of the claims 26 to 28 or 30, wherein the nucleotide sequence of Kir6.2 carries an A at position 1009 with respect to SEQ ID No. 2 of the coding sequence.

32. Use according to one of the claims 26 to 28, 30 or 31, wherein the test kit contain a primer set able to amplify a polynucleotide fragment comprising position 67 with respect to SEQ ID No. 2 and/or 1009 with respect to SEQ ID No. 2 of the Kir6.2 coding sequence.

33. Use according to one of the claim 32, wherein the test kit contains at least one of the primers according to SEQ ID Nos. 9 to 12.

34. Use according to one of the claims 26 to 28 or 30 to 32, wherein the test kit contains a primer set able to amplify a polynucleotide fragment comprising positions 606/607/680 with respect to SEQ ID No. 13 and/or 1478/79/80 with respect to SEQ ID No. 13 of the Kir6.2 genomic sequence according to SEQ ID No. 13.

35. Use according to one of the claim 34, wherein the test kit contains at least one of the primers according to SEQ ID No. 9 to 12.

36. Use according to one of the claims 26 to 28, 30 or 32, wherein the test kit contains one or more nucleic acid probes specifically recognizing Kir6.2 genomic DNA or RNA with a nucleotide sequence coding for another amino acid than Glutaminic Acid at position 23 with respect to SEQ ID No. 1 and/or Valine at position 337 with respect to SEQ ID No. 1 under stringent conditions.

## Patentansprüche

1. Verfahren zur Identifizierung eines erhöhten Risikos von Angina pectoris bei einem zuckerkranken Patienten, wobei man das Vorliegen einer anderen Aminosäure als Glutaminsäure in Position 23 und/oder das Vorliegen einer anderen Aminosäure als Valin in Position 337 im Kir6.2-Protein in einer Probe bestimmt.

2. Verfahren zur Identifizierung eines erhöhten Risikos von Angina pectoris bei einem zuckerkranken Patienten, wobei man das Vorliegen einer Variation in der Kir6.2-Nukleotidsequenz auf einem oder beiden Allelen des Kir6.2-Gens, was zu einer Kir6.2-Polypeptidsequenz führt, die eine andere Aminosäure als Glutaminsäure in Position 23 und/oder eine andere Aminosäure als Valin in Position 337 im Kir6.2-Protein enthält, in einer Probe bestimmt.

3. Verfahren zur Anpassung der Dosierung eines zur Behandlung und/oder Vorbeugung von Angina pectoris bei einem zuckerkranken Patienten geeigneten Pharmazeutikums, wobei man
I. das Vorliegen eines Kir6.2 mit
a) einer anderen Aminosäure als Glutaminsäure in Position 23 und/oder einer anderen Aminosäure als Valin in Position 337 im Kir6.2-Protein,
b) einer Variation in der Nukleotidsequenz auf einem oder beiden Allelen des Kir6.2-Gens, was zu einer anderen Aminosäure als Glutaminsäure in Position 23 und/oder einer anderen Aminosäure als Valin in Position 337 im Kir6.2-Protein führt, oder
c) Kir6.2-23-KK und/oder Kir6.2-337-II in einer Probe eines zuckerkranken Patienten bestimmt.

4. Verfahren oder Verwendung nach einem der Ansprüche 1 bis 3, wobei es sich bei der Aminosäure in Position 23 in Bezug auf SEQ ID Nr. 1 des Kir6.2-Proteins um Lys handelt.

5. Verfahren oder Verwendung nach einem der Ansprüche 1 bis 4, wobei es sich bei der Aminosäure in Position 337 in Bezug auf SEQ ID Nr. 1 des Kir6.2-Proteins um Ile handelt.

6. Verfahren oder Verwendung nach einem der Ansprüche 2 bis 5, wobei die Nukleotidsequenz ein A in Position 67 in Bezug auf SEQ ID Nr. 2 der Kir6.2 codierenden Sequenz trägt.

7. Verfahren oder Verwendung nach einem der Ansprüche 2 bis 6, wobei die Nukleotidsequenz ein G in Position 1009 in Bezug auf SEQ ID Nr. 2 der Kir6.2 codierenden Sequenz trägt.

8. Verfahren oder Verwendung nach einem der vorhergehenden Ansprüche 1 bis 7, wobei es sich bei dem Diabetes um Diabetes mellitus handelt.

9. Verfahren nach einem der vorhergehenden Ansprüche 1 bis 8, wobei das Vorliegen von Kir6.2-23-KK und/oder Kir6.2-337-II in der Probe bestimmt wird.

10. Verfahren nach einem der vorhergehenden Ansprüche 1 bis 9, wobei es sich bei der Probe um eine histologische Probe, einen Zell- oder Gewebeextrakt oder eine Zelle handelt.

11. Verfahren nach einem der Ansprüche 1 bis 10, wobei die Probe menschlichen Ursprungs ist.

12. Verfahren nach einem der Ansprüche 1 bis 11, wobei das Vorliegen des Aminosäureaustauschs durch
a) Amplifikation eines Polynukleotidfragments aus einer genomische DNA enthaltenden biologischen Probe, wobei die chromosomale DNA vorzugsweise mittels einer PCR-Reaktion unter Verwendung von Primern, mit denen ein Polynukleotidfragment, das diejenigen Nukleotidpositionen der genomischen Kir6.2-Sequenz, die Position 67/68/69 und/oder 1009/1010/1011 in Bezug auf SEQ ID Nr. 2 der codierenden Sequenz entsprechen, umfasst, amplifiziert werden kann, aus der Probe isoliert wurde,
b) Sequenzieren des Polynukleotidfragments gemäß a) bestimmt wird.

13. Verfahren nach Anspruch 12, wobei die Amplifikation des Polynukleotidfragments unter Verwendung wenigstens eines und vorzugsweise von zwei der Primer gemäß SEQ ID Nr. 9 bis 12 erfolgt.

14. Verfahren nach Anspruch 12 oder 13, wobei die Sequenzierreaktion unter Verwendung eines der Primer gemäß SEQ ID Nr. 9 bis 12 erfolgt.

15. Verfahren nach einem der Ansprüche 1 bis 11, wobei das Vorliegen des Aminosäureaustauschs durch
a) Isolieren genomischer DNA aus einer genomische DNA enthaltenden biologischen Probe,
b) Immobilisieren derselben auf einem Träger,
c) Hybridisieren einer oder mehrerer Sonden, die an die einen oder mehrere, zu einem der oder beiden Aminosäureaustausche führende Nukleotidaustausche enthaltende Kir6.2-Sequenz mit höherer Affinität als an die Kir6.2-Wildtypsequenz binden können, an die immobilisierte DNA unter stringenten Bedingungen, bestimmt wird.

16. Verfahren nach Anspruch 15, wobei die isolierte genomische DNA
a) auf einer Chip-Matrix, einer geeigneten Membran immobilisiert oder
b) auf eine Gelmatrix überführt und mittels Blotting auf eine geeignete Membran übertragen wird.

17. Verfahren nach einem der Ansprüche 1 bis 11, wobei der Aminosäureaustausch durch
a) Amplifikation eines Polynukleotidfragments mittels einer RT-PCR-Reaktion unter Verwendung von Primern, mit denen ein Polynukleotidfragment, das Position 67 in Bezug auf SEQ ID Nr. 2 und/oder 1009 in Bezug auf SEQ ID Nr. 2 der Kir6.2-cDNA-Sequenz umfasst, amplifiziert werden kann, sowie unter Verwendung einer RNA aus einem Individuum enthaltenden biologischen Probe und vorzugsweise unter Verwendung isolierter RNA aus der Probe,
b) Sequenzieren des Polynukleotidfragments gemäß a) bestimmt wird.

18. Verfahren nach Anspruch 17, wobei wenigstens einer der Primer gemäß SEQ ID Nr. 9 bis 12 verwendet wird.

19. Verfahren nach einem der Ansprüche 1 bis 11, wobei der Aminosäureaustausch durch
a) Isolieren von RNA aus einer RNA aus einem Individuum enthaltenden biologischen Probe,
b) Immobilisieren derselben auf einem Träger,
c) Hybridisieren der immobilisierten RNA an eine -oder mehrere Sonden, die unter stringenten Bedingungen an für ein Kir6.2-Polypeptid, das eine andere Aminosäure als Glutaminsäure in Position 23 in Bezug auf SEQ ID Nr. 1 und/oder Valin in Position 337 in Bezug auf SEQ ID Nr. 1 des Kir6.2-Proteins enthält, codierende Kir6.2-RNA mit höherer Affinität als an für ein Kir6.2-Polypeptid, das Glutaminsäure in Position 23 in Bezug auf SEQ ID Nr. 1 und/oder Valin in Position 337 in Bezug auf SEQ ID Nr. 1 enthält, codierende Kir6.2-RNA binden können, bestimmt wird.

20. Verfahren nach Anspruch 19, wobei die isolierte RNA
a) auf einer Chip-Matrix oder einer geeigneten Membran immobilisiert oder
b) auf eine Gelmatrix überführt und anschließend mittels Blotting auf eine geeignete Membran übertragen wird.

21. Verfahren nach einem der Ansprüche 1 bis 11, wobei der Aminosäureaustausch durch
a) Isolieren von Protein aus einer Proteine aus einem Individuum enthaltenden biologischen Probe,
b) Immobilisieren desselben auf einem Träger,
c) Ausführen einer Bindungsreaktion mit einem Antikörper, der an Kir6.2, das eine andere Aminosäure als Glutaminsäure in Position 23 in Bezug auf SEQ ID Nr. 1 und/oder als Valin in Position 337 in Bezug auf SEQ ID Nr. 1 seiner Polypeptidkette enthält, mit höherer Affinität als an ein Kir6.2, das Glutaminsäure in Position 337 in Bezug auf SEQ ID Nr. 1 und/oder Valin in Position 337 in Bezug auf SEQ ID Nr. 1 seiner Polypeptidkette enthält, binden kann,
d) Sichtbarmachen der Bindung des Antikörpers an das Protein bestimmt wird.

22. Verfahren nach Anspruch 21, wobei das isolierte Protein
a) auf einer Chip-Matrix oder einer geeigneten Membran immobilisiert,
b) auf eine Gelmatrix überführt und anschließend auf einer geeigneten Membran immobilisiert oder
c) auf einer ELISA-Platte immobilisiert wird.

23. Verfahren nach einem der Ansprüche 1 bis 11, wobei der Aminosäureaustausch durch
a) Ausführen einer Bindungsreaktion an einer histologischen Probe aus einem Individuum mit einem Antikörper, der an Kir6.2, das eine andere Aminosäure als Glutaminsäure in Position 23 in Bezug auf SEQ ID Nr. 1 und/oder Valin in Position 337 in Bezug auf SEQ ID Nr. 1 seiner Polypeptidkette enthält, mit höherer Affinität als an ein Kir6.2, das Glutaminsäure in Position 337 in Bezug auf SEQ ID Nr. 1 und/oder Valin in Position 337 in Bezug auf SEQ ID Nr. 1 seiner Polypeptidkette enthält, binden kann,
b) Sichtbarmachen der Bindung des Antikörpers an das Protein bestimmt wird.

24. Verfahren nach Anspruch 23, wobei der Nachweis der Bindung mittels einer immunhistochemischen oder immunradiologischen Reaktion erfolgt.

25. Verwendung eines Testkits zum Testen des Vorliegens einer anderen Aminosäure als Glutaminsäure in Position 23 in Bezug auf SEQ ID Nr. 1 und/oder einer anderen Aminosäure als Valin in Position 337 in Bezug auf SEQ ID Nr. 1 im Kir6.2-Protein, Kir6.2-23-KK oder Kir6.2-337-II, wobei der Kit wenigstens ein Mittel zum Nachweis dieses Vorliegens im Protein enthält, um ein erhöhtes Risiko von Angina pectoris bei einem zuckerkranken Patienten zu identifizieren.

26. Verwendung eines Testkits zum Testen des Vorliegens einer Variation in der Kir6.2-Nukleotidsequenz auf einem oder beiden Allelen des Kir6.2-Gens, was zu einer Kir6.2-Polypeptidsequenz führt, die eine andere Aminosäure als Glutaminsäure in Position 23 in Bezug auf SEQ ID Nr. 1 und/oder eine andere Aminosäure als Valin in Position 337 in Bezug auf SEQ ID Nr. 1 im Kir6.2-Protein enthält, wobei der Kit wenigstens ein Mittel zum Nachweis dieser Variation in der Nukleotidsequenz enthält, um ein erhöhtes Risiko von Angina pectoris bei einem zuckerkranken Patienten zu identifizieren.

27. Verwendung nach einem der Ansprüche, 25 oder 26,
wobei es sich bei der Aminosäure in Position 23 in Bezug auf SEQ ID Nr. 1 um Lys handelt.

28. Verwendung nach einem der Ansprüche 25 bis 27, wobei es sich bei der Aminosäure in Position 337 in Bezug auf SEQ ID Nr. 1 um Ile handelt.

29. Verwendung nach einem der Ansprüche 25, 27 oder 28,
wobei der Testkit einen Antikörper enthält, der in seinen Bindungseigenschaften zwischen Glutaminsäure in Position 23 in Bezug auf SEQ ID Nr. 1 und/oder Valin in Position 337 in Bezug auf SEQ ID Nr. 1 enthaltendem Kir6.2 unterscheidet.

30. Verwendung nach einem der Ansprüche 26 bis 28, wobei die Nukleotidsequenz von Kir6.2 ein G in Position 67 in Bezug auf SEQ ID Nr. 2 der codierenden Sequenz trägt.

31. Verwendung nach einem der Ansprüche 26 bis 28 oder 30, wobei die Nukleotidsequenz von Kir6.2 ein A in Position 1009 in Bezug auf SEQ ID Nr. 2 der codierenden Sequenz trägt.

32. Verwendung nach einem der Ansprüche 26 bis 28, 30 oder 31, wobei der Testkit einen Primersatz enthält, mit dem ein Polynukleotidfragment, das Position 67 in Bezug auf SEQ ID Nr. 2 und/oder 1009 in Bezug auf SEQ ID Nr. 2 der Kir6.2 codierenden Sequenz umfasst, amplifiziert werden kann.

33. Verwendung nach Anspruch 32, wobei der Testkit wenigstens einen der Primer gemäß SEQ ID Nr. 9 bis 12 enthält.

34. Verwendung nach einem der Ansprüche 26 bis 28 oder 30 bis 32, wobei der Testkit' einen Primersatz enthält, mit dem ein Polynukleotidfragment, das Position 606/607/680 in Bezug auf SEQ ID Nr. 13 und/oder 1478/79/80 in Bezug auf SEQ ID Nr. 13 der genomischen Kir6.2-Sequenz gemäß SEQ ID Nr. 13 umfasst, amplifiziert werden kann.

35. Verwendung nach Anspruch 34, wobei der Testkit wenigstens einen der Primer gemäß SEQ ID Nr. 9 bis 12 enthält.

36. Verwendung nach einem der Ansprüche 26 bis 28, 30 oder 32, wobei der Testkit eine oder mehrere Nukleinsäuresonden enthält, die genomische Kir6.2-DNA oder -RNA mit einer Nukleotidsequenz, die für eine andere Aminosäure als Glutaminsäure in Position 23 in Bezug auf SEQ ID Nr. 1 und/oder Valin in Position 337 in Bezug auf SEQ ID Nr. 1 codiert, unter stringenten Bedingungen spezifisch erkennen.

## Revendications

1. Procédé d'identification d'une augmentation du risque d'angine de poitrine chez un patient diabétique, **caractérisé en ce que** la présence d'un acide aminé autre que l'acide glutamique à la position 23 et/ou la présence d'un acide aminé autre que la valine à la position 337 dans la protéine Kir6.2 est déterminée dans un échantillon.

2. Procédé d'identification d'une augmentation du risque d'angine de poitrine chez un patient diabétique, **caractérisé en ce que** la présence d'une variation dans la séquence nucléotidique Kir6.2 sur l'un ou les deux allèles du gène Kir6.2 conduisant à une séquence polypeptidique Kir6.2 comportant un acide aminé autre que l'acide glutamique à la position 23 et/ou un acide aminé autre que la valine à la position 337 dans la protéine Kir6.2 est déterminée dans un échantillon.

3. Procédé d'adaptation de la posologie d'un produit pharmaceutique utile pour traiter et/ou prévenir l'angine de poitrine chez un patient diabétique,
**caractérisé en ce que** :
I. la présence de Kir6.2 avec
a) un acide aminé autre que l'acide glutamique à la position 23 et/ou un acide aminé autre que la valine à la position 337 dans la protéine Kir6.2
b) une variation dans la séquence nucléotidique sur l'un ou les deux allèles du gène Kir6.2 conduisant à un acide aminé autre que l'acide glutamique à la position 23 et/ou un acide aminé autre que la valine à la position 337 dans la protéine Kir6.2 ou
c) Kir6.2-23-KK et/ou Kir6.2-337-II est déterminée dans un échantillon d'un patient diabétique.

4. Procédé ou utilisation selon une des revendications 1 à 3, **caractérisé en ce que** l'acide aminé à la position 23 en référence à SEQ ID N° 1 de la protéine Kir6.2 est Lys.

5. Procédé ou utilisation selon une des revendications 1 à 4, **caractérisé en ce que** l'amino à la position 337 en référence à SEQ ID N°1 de la protéine Kir6.2 est Ile.

6. Procédé ou utilisation selon une des revendications 2 à 5, **caractérisé en ce que** la séquence nucléotidique comporte un A à la position 67 en référence à SEQ ID N° 2 de la séquence codante de Kir6.2.

7. Procédé ou utilisation selon une des revendications 2 à 6, **caractérisé en ce que** la séquence nucléotidique comporte un G à la position 1009 en référence à SEQ ID N° 2 de la séquence codante de Kir6.2.

8. Procédé ou utilisation selon une des revendications précédentes 1 à 7, **caractérisé en ce que** le diabète est le diabète sucré.

9. Procédé ou utilisation selon une des revendications précédentes 1 à 8, **caractérisé en ce que** la présence de Kir6.2-23-KK et/ou Kir6.2-337-II est déterminée dans l'échantillon.

10. Procédé selon une des revendications précédentes 1 à 9, **caractérisé en ce que** l'échantillon est un échantillon histologique, un extrait de cellules ou de tissu ou une cellule.

11. Procédé selon une des revendications 1 à 10,
**caractérisé en ce que** l'échantillon est d'origine humaine.

12. Procédé selon une des revendications 1 à 11,
**caractérisé en ce que** la présence de la substitution d'acide aminé est déterminée par
a) amplification d'un fragment de polynucléotide d'un échantillon biologique contenant de l'ADN génomique, dans lequel l'ADN chromosomique a de préférence été isolé à partir de l'échantillon, au moyen d'une réaction PCR en utilisant des amorces capables d'amplifier un fragment de polynucléotide comprenant les positions de nucléotide de la séquence Kir6.2 génomique qui correspondent aux positions 67/68/69 et/ou 1009/1010/1011 en référence à SEQ ID N° 2 de la séquence codante ;
b) séquençage du fragment de polynucléotide selon a).

13. Procédé selon la revendication 12, **caractérisé en ce que** l'amplification du fragment de polynucléotide est effectuée en utilisant au moins une et de préférence deux des amorces selon SEQ ID N° 9 à 12.

14. Procédé selon la revendication 12 ou 13,
**caractérisé en ce que** la réaction de séquençage est effectuée en utilisant une des amorces selon SEQ ID N° 9 à 12.

15. Procédé selon une des revendications 1 à 11,
**caractérisé en ce que** la présence de la substitution d'acide aminé est déterminée par
a) isolement d'ADN génomique à partir d'un échantillon biologique contenant de l'ADN génomique,
b) immobilisation de celui-ci sur un support,
c) hybridation de l'ADN immobilisé avec une ou plusieurs sondes capables de se lier à la séquence Kir6.2 comportant une ou plusieurs substitutions de nucléotide conduisant à l'une ou les deux substitutions d'acide aminé ayant une plus grande affinité que pour la séquence Kir6.2 de type sauvage dans des conditions stringentes.

16. Procédé selon la revendication 15, **caractérisé en ce que** l'ADN génomique isolé est
a) immobilisé sur une matrice de puce, une membrane adaptée ou
b) transféré sur une matrice de gel et transféré sur une membrane adaptée.

17. Procédé selon une des revendications 1 à 11,
**caractérisé en ce que** la substitution d'acide aminé est déterminée par
a) amplification d'un fragment de polynucléotide au moyen d'une réaction RT-PCR en utilisant des amorces capables d'amplifier un fragment de polynucléotide comprenant les positions 67 en référence à SEQ ID N° 2 et/ou 1009 en référence à SEQ ID N° 2 de la séquence d'ADNc Kir6.2 et en utilisant un échantillon biologique contenant de l'ARN d'un individu et, de préférence, en utilisant de l'ARN isolé à partir de l'échantillon,
b) séquençage du fragment de polynucléotide selon a).

18. Procédé selon la revendication 17, **caractérisé en ce qu'**au moins une des amorces selon SEQ ID N° 9 à 12 est utilisée.

19. Procédé selon une des revendications 1 à 11,
**caractérisé en ce que** la substitution d'acide aminé est déterminée par
a) isolement d'ARN à partir d'un échantillon biologique contenant de l'ARN d'un individu,
b) immobilisation de celui-ci sur un support,
c) hybridation de l'ARN immobilisé avec une ou plusieurs sondes capables de se lier dans des conditions stringentes à l'ARN Kir6.2 codant pour un polypeptide Kir6.2 comportant un acide aminé autre que l'acide glutamique à la position 23 en référence à SEQ ID N° 1 et/ou la valine à la position 337 en référence à SEQ ID N° 1 de la protéine Kir6.2 avec une plus grande affinité que pour l'ARN Kir6.2 codant pour un polypeptide Kir6.2 comportant l'acide glutamique à la position 23 en référence à SEQ ID N° 1 et/ou la valine à la position 337 en référence à SEQ ID N° 1.

20. Procédé selon la revendication 19, **caractérisé en ce que** l'ARN isolé est
a) immobilisé sur une matrice de puce ou une membrane adaptée, ou
b) transféré sur une matrice de gel et ensuite transféré sur une membrane adaptée.

21. Procédé selon une des revendications 1 à 11,
**caractérisé en ce que** la substitution d'acide aminé est déterminée par
a) isolement des protéines d'un échantillon biologique contenant des protéines d'un individu ;
b) immobilisation de celles-ci sur un support ;
c) conduite d'une réaction de liaison avec un anticorps capable de se lier avec une plus grande affinité à Kir6.2 comportant un acide aminé autre que l'acide glutamique à la position 23 en référence à SEQ ID N° 1 et/ou que la valine à la position 337 en référence à SEQ ID N° 1 de sa chaîne polypeptidique que Kir6.2 comportant l'acide glutamique à la position 337 en référence à SEQ ID N° 1 et/ou la valine à la position 337 en référence à SEQ ID N° 1 de sa chaîne polypeptidique ;
d) rendre visible la liaison de l'anticorps à la protéine.

22. Procédé selon la revendication 21 **caractérisé en ce que** la protéine isolée est
a) immobilisée sur une matrice de puce ou une membrane adaptée ;
b) transférée sur une matrice de gel et ensuite immobilisée sur une membrane adaptée, ou
c) immobilisée sur une plaque ELISA.

23. Procédé selon une des revendications 1 à 11,
**caractérisé en ce que** la substitution d'acide aminé est déterminée par
a) conduite sur un échantillon histologique d'un individu d'une réaction de liaison avec un anticorps capable de se lier avec une plus grande affinité à Kir6.2 comportant un acide aminé autre que l'acide glutamique à la position 23 en référence à SEQ ID N° 1 et/ou la valine à la position 337 en référence à SEQ ID N° 1 de sa chaîne polypeptidique qu'à Kir6.2 comportant l'acide glutamique à la position 337 en référence à SEQ ID N° 1 et/ou la valine à la position 337 en référence à SEQ ID N° 1 de sa chaîne polypeptidique ;
b) rendre visible la liaison de l'anticorps à la protéine.

24. Procédé selon la revendication 23, **caractérisé en ce que** la détection de la liaison est effectuée au moyen d'une réaction immunohistochimique ou immunoradiologique.

25. Utilisation d'un kit d'essai pour déterminer la présence d'un acide aminé autre que l'acide glutamique à la position 23 en référence à SEQ ID N° 1 et/ou d'un acide aminé autre que la valine à la position 337 en référence à SEQ ID N° 1 dans la protéine Kir6.2, Kir6.2-23-KK ou Kir6.2-337-II, **caractérisé en ce que** le kit contient au moins un moyen pour la détection de ladite présence dans la protéine, afin d'identifier une augmentation du risque d'angine de poitrine chez un patient diabétique.

26. Utilisation d'un kit d'essai pour déterminer la présence d'une variation dans la séquence nucléotidique Kir6.2 sur l'un ou les deux allèles du gène Kir6.2 conduisant à une séquence polypeptidique Kir6.2 comportant un acide aminé autre que l'acide glutamique à la position 23 en référence à SEQ ID N° 1 et/ou un acide aminé autre que la valine à la position 337 en référence à SEQ ID N° 1 dans la protéine Kir6.2, **caractérisée en ce que** le kit contient au moins un moyen pour la détection de ladite variation dans la séquence nucléotidique, afin d'identifier une augmentation du risque d'angine de poitrine chez un patient diabétique.

27. Utilisation selon une des revendications 25 ou 26 **caractérisée en ce que** l'acide aminé à la position 23 en référence à SEQ ID N° 1 est Lys.

28. Utilisation selon une des revendications 25 à 27, **caractérisée en ce que** l'acide aminé à la position 337 en référence à SEQ ID N° 1 est Ile.

29. Utilisation selon une des revendications 25, 27 ou 28, **caractérisée en ce que** le kit d'essai contient un anticorps discriminant par ses caractéristiques de liaison entre Kir6.2 comportant l'acide glutamique à la position 23 en référence à SEQ ID N° 1 et/ou la valine à la position 337 en référence à SEQ ID N° 1.

30. Utilisation selon une des revendications 26 à 28, **caractérisée en ce que** la séquence nucléotidique de Kir6.2 comporte un G à la position 67 en référence à SEQ ID N° 2 de la séquence codante.

31. Utilisation selon une des revendications 26 à 28 ou 30, **caractérisée en ce que** la séquence nucléotidique de Kir6.2 comporte un A à la position 1009 en référence à SEQ ID N° 2 de la séquence codante.

32. Utilisation selon une des revendications 26 à 28, 30 ou 31, **caractérisée en ce que** le kit d'essai contient un jeu d'amorces capables d'amplifier un fragment de polynucléotide comprenant la position 67 en référence à SEQ ID N° 2 et/ou 1009 en référence à SEQ ID N° 2 de la séquence codante de Kir6.2.

33. Utilisation selon la revendication 32, **caractérisée en ce que** le kit d'essai contient au moins une des amorces selon SEQ ID N° 9 à 12.

34. Utilisation selon une des revendications 26 à 28 ou 30 à 32, **caractérisée en ce que** le kit d'essai contient un jeu d'amorces capables d'amplifier un fragment de polynucléotide comprenant les positions 606/607/680 en référence à SEQ ID N° 13 et/ou 1478/79/80 en référence à SEQ ID N° 13 de la séquence génomique de Kir6.2 selon SEQ ID N° 13.

35. Utilisation selon la revendication 34, **caractérisée en ce que** le kit d'essai contient au moins une des amorces selon SEQ ID N° 9 à 12.

36. Utilisation selon une des revendications 26 à 28, 30 ou 32, **caractérisée en ce que** le kit d'essai contient une ou plusieurs sondes d'acide nucléique reconnaissant spécifiquement l'ADN génomique ou l'ARN de Kir6.2 ayant une séquence nucléotidique codant pour un acide aminé autre que l'acide glutamique à la position 23 en référence à SEQ ID N° 1 et/ou la valine à la position 337 en référence à SEQ ID N° 1 dans des conditions stringentes.


### SEQUENCE LISTING

<110> Aventis Pharma Deutschland GmbH
<120> Association of polymorphisms with coronary heart disease
<130> DEAV2004/0064
<140> 04021528.7
   <141> 2004-09-10
<160> 13
<170> PatentIn ver. 2.1
<210> 1
   <211> 390
   <212> PRT
   <213> Homo sapiens
<400> 1
   Met Leu Ser Arg Lys Gly Ile Ile Pro Glu Glu Tyr Val Leu Thr Arg
    1               5                  10                  15
   Leu Ala Glu Asp Pro Ala Glu Pro Arg Tyr Arg Ala Arg Gln Arg Arg
               20                  25                  30
   Ala Arg Phe Val Ser Lys Lys Gly Asn Cys Asn Val Ala His Lys Asn
           35                  40                  45
   Ile Arg Glu Gln Gly Arg Phe Leu Gln Asp Val Phe Thr Thr Leu Val
       50                  55                  60
   Asp Leu Lys Trp Pro His Thr Leu Leu Ile Phe Thr Met Ser Phe Leu
   65                  70                  75                  80
   Cys Ser Trp Leu Leu Phe Ala Met Ala Trp Trp Leu Ile Ala Phe Ala
                   85                  90                  95
   His Gly Asp Leu Ala Pro Ser Glu Gly Thr Ala Glu Pro Cys Val Thr
               100                 105                 110
   Ser Ile His Ser Phe Ser Ser Ala Phe Leu Phe Ser Ile Glu Val Gln
           115                 120                 125
   Val Thr Ile Gly Phe Gly Gly Arg Met Val Thr Glu Glu Cys Pro Leu
       130                 135                 140
   Ala Ile Leu Ile Leu Ile Val Gln Asn Ile Val Gly Leu Met Ile Asn
   145                 150                 155                 160
   Ala Ile Met Leu Gly Cys Ile Phe Met Lys Thr Ala Gln Ala His Arg
                   165                 170                 175
   Arg Ala Glu Thr Leu Ile Phe Ser Lys His Ala Val Ile Ala Leu Arg
               180                 185                 190
   His Gly Arg Leu Cys Phe Met Leu Arg Val Gly Asp Leu Arg Lys Ser
           195                 200                 205
   Met Ile Ile Ser Ala Thr Ile His Met Gln Val Val Arg Lys Thr Thr
       210                 215                 220
   Ser Pro Glu Gly Glu Val Val Pro Leu His Gln Val Asp Ile Pro Met
   225                 230                 235                 240
   Glu Asn Gly Val Gly Gly Asn Ser Ile Phe Leu Val Ala Pro Leu Ile
   245 250 255
   Ile Tyr His Val Ile Asp Ala Asn Ser Pro Leu Tyr Asp Leu Ala Pro
   260 265 270
   Ser Asp Leu His His His Gln Asp Leu Glu Ile Ile Val Ile Leu Glu
   275 280 285
   Gly Val Val Glu Thr Thr Gly Ile Thr Thr Gln Ala Arg Thr Ser Tyr
   290 295 300
   Leu Ala Asp Glu Ile Leu Trp Gly Gln Arg Phe Val Pro Ile Val Ala
   305 310 315 320
   Glu Glu Asp Gly Arg Tyr Ser Val Asp Tyr Ser Lys Phe Gly Asn Thr
   325 330 335
   Val Lys Val Pro Thr Pro Leu Cys Thr Ala Arg Gln Leu Asp Glu Asp
   340 345 350
   His Ser Leu Leu Glu Ala Leu Thr Leu Ala Ser Ala Arg Gly Pro Leu
   355 360 365
   Arg Lys Arg Ser Val Pro Met Ala Lys Ala Lys Pro Lys Phe Ser Ile
   370 375 380
   Ser Pro Asp Ser Leu Ser
   385 390
<210> 2
   <211> 1173
   <212> DNA
   <213> Homo sapiens
<400> 2
   atgctgtccc gcaagggcat catccccgag gaatacgtgc tgacacgcct ggcagaggac 60
   cctgccgagc ccaggtaccg tgcccgccag cggagggccc gctttgtgtc caagaaaggc 120
   aactgcaacg tggcccacaa gaacatccgg gagcagggcc gcttcctgca ggacgtgttc 180
   accacgctgg tggacctcaa gtggccacac acattgctca tcttcaccat gtccttcctg 240
   tgcagctggc tgctcttcgc catggcctgg tggctcatcg ccttcgccca cggtgacctg 300
   gcccccagcg agggcactgc tgagccctgt gtcaccagca tccactcctt ctcgtctgcc 360
   ttcctttcct ccattgaggt ccaagtgact attggctttg gggggcgcat ggtgactgag 420
   gagtgcccac tggccatcct gatcctcatc gtgcagaaca tcgtggggct catgatcaac 480
   gccatcatgc ttggctgcat cttcatgaag actgcccaag cccaccgcag ggctgagacc 540
   ctcatcttca gcaagcatgc ggtgatcgcc ctgcgccacg gccgcctctg cttcatgcta 600
   cgtgtgggtg acctccgcaa gagcatgatc atcagcgcca ccatccacat gcaggtggta 660
   cgcaagacca ccagccccga gggcgaggtg gtgcccctcc accaggtgga catccccatg 720
   gagaacggcg tgggtggcaa cagcatcttc ctggtggccc cgctgatcat ctaccatgtc 780
   attgatgcca acagcccact ctacgacctg gcacccagcg acctgcacca ccaccaggac 840
   ctcgagatca tcgtcatcct ggaaggcgtg gtggaaacca cgggcatcac cacccaggcc 900
   cgcacctcct acctggccga tgagatcctg tggggccagc gctttgtgcc cattgtagct 960
   gaggaggacg gacgttactc tgtggactac tccaagtttg gcaacaccgt caaagtgccc 1020
   acaccactct gcacggcccg ccagcttgat gaggaccaca gcctactgga agctctgacc 1080
   ctcgcctcag cccgcgggcc cctgcgcaag cgcagcgtgc ccatggccaa ggccaagccc 1140
   aagttcagca tctctccaga ttccctgtcc tga 1173
<210> 3
   <211> 390
   <212> PRT
   <213> Homo sapiens
<400> 3
   Met Leu Ser Arg Lys Gly Ile Ile Pro Glu Glu Tyr Val Leu Thr Arg
   1               5                   10                  15
   Leu Ala Glu Asp Pro Ala Lys Pro Arg Tyr Arg Ala Arg Gln Arg Arg
   20 25 30
   Ala Arg Phe Val Ser Lys Lys Gly Asn Cys Asn Val Ala His Lys Asn
   35 40 45
   Ile Arg Glu Gln Gly Arg Phe Leu Gln Asp Val Phe Thr Thr Leu Val
   50 55 60
   Asp Leu Lys Trp Pro His Thr Leu Leu Ile Phe Thr Met Ser Phe Leu
   65 70 75 80
   Cys Ser Trp Leu Leu Phe Ala Met Ala Trp Trp Leu Ile Ala Phe Ala
   85 90 95
   His Gly Asp Leu Ala Pro Ser Glu Gly Thr Ala Glu Pro Cys Val Thr
   100 105 110
   Ser Ile His Ser Phe Ser Ser Ala Phe Leu Phe Ser Ile Glu Val Gln
   115 120 125
   Val Thr Ile Gly Phe Gly Gly Arg Met Val Thr Glu Glu Cys Pro Leu
   130 135 140
   Ala Ile Leu Ile Leu Ile Val Gln Asn Ile Val Gly Leu Met Ile Asn
   145 150 155 160
   Ala Ile Met Leu Gly Cys Ile Phe Met Lys Thr Ala Gln Ala His Arg
   165 170 175
   Arg Ala Glu Thr Leu Ile Phe Ser Lys His Ala Val Ile Ala Leu Arg
   180 185 190
   His Gly Arg Leu Cys Phe Met Leu Arg Val Gly Asp Leu Arg Lys Ser
   195 200 205
   Met Ile Ile Ser Ala Thr Ile His Met Gln Val Val Arg Lys Thr Thr
   210 215 220
   Ser Pro Glu Gly Glu Val Val Pro Leu His Gln Val Asp Ile Pro Met
   225 230 235 240
   Glu Asn Gly Val Gly Gly Asn Ser Ile Phe Leu Val Ala Pro Leu Ile
   245 250 255
   Ile Tyr His Val Ile Asp Ala Asn Ser Pro Leu Tyr Asp Leu Ala Pro
   260 265 270
   Ser Asp Leu His His His Gln Asp Leu Glu Ile Ile Val Ile Leu Glu
   275 280 285
   Gly Val Val Glu Thr Thr Gly Ile Thr Thr Gln Ala Arg Thr Ser Tyr
   290 295 300
   Leu Ala Asp Glu Ile Leu Trp Gly Gln Arg Phe Val Pro Ile Val Ala
   305 310 315 320
   Glu Glu Asp Gly Arg Tyr Ser Val Asp Tyr Ser Lys Phe Gly Asn Thr
   325 330 335
   Val Lys Val Pro Thr Pro Leu Cys Thr Ala Arg Gln Leu Asp Glu Asp
   340 345 350
   His Ser Leu Leu Glu Ala Leu Thr Leu Ala Ser Ala Arg Gly Pro Leu
   355 360 365
   Arg Lys Arg Ser Val Pro Met Ala Lys Ala Lys Pro Lys Phe Ser Ile
   370 375 380
   Ser Pro Asp Ser Leu Ser
   385 390
<210> 4
   <211> 1173
   <212> DNA
   <213> Homo sapiens
<400> 4
   atgctgtccc gcaagggcat catccccgag gaatacgtgc tgacacgcct ggcagaggac 60
   cctgccaagc ccaggtaccg tgcccgccag cggagggccc gctttgtgtc caagaaaggc 120
   aactgcaacg tggcccacaa gaacatccgg gagcagggcc gcttcctgca ggacgtgttc 180
   accacgctgg tggacctcaa gtggccacac acattgctca tcttcaccat gtccttcctg 240
   tgcagctggc tgctcttcgc catggcctgg tggctcatcg ccttcgccca cggtgacctg 300
   gcccccagcg agggcactgc tgagccctgt gtcaccagca tccactcctt ctcgtctgcc 360
   ttccttttct ccattgaggt ccaagtgact attggctttg gggggcgcat ggtgactgag 420
   gagtgcccac tggccatcct gatcctcatc gtgcagaaca tcgtggggct catgatcaac 480
   gccatcatgc ttggctgcat cttcatgaag actgcccaag cccaccgcag ggctgagacc 540
   ctcatcttca gcaagcatgc ggtgatcgcc ctgcgccacg gccgcctctg cttcatgcta 600
   cgtgtgggtg acctccgcaa gagcatgatc atcagcgcca ccatccacat gcaggtggta 660
   cgcaagacca ccagccccga gggcgaggtg gtgcccctcc accaggtgga catccccatg 720
   gagaacggcg tgggtggcaa cagcatcttc ctggtggccc cgctgatcat ctaccatgtc 780
   attgatgcca acagcccact ctacgacctg gcacccagcg acctgcacca ccaccaggac 840
   ctcgagatca tcgtcatcct ggaaggcgtg gtggaaacca cgggcatcac cacccaggcc 900
   cgcacctcct acctggccga tgagatcctg tggggccagc gctttgtgcc cattgtagct 960
   gaggaggacg gacgttactc tgtggactac tccaagtttg gcaacaccgt caaagtgccc 1020
   acaccactct gcacggcccg ccagcttgat gaggaccaca gcctactgga agctctgacc 1080
   ctcgcctcag cccgcgggcc cctgcgcaag cgcagcgtgc ccatggccaa ggccaagccc 1140
   aagttcagca tctctccaga ttccctgtcc tga                              1173
<210> 5
   <211> 390
   <212> PRT
   <213> Homo sapiens
<400> 5
<210> 6
   <211> 1635
   <212> DNA
   <213> Homo sapiens
<400> 6
   ctgaggctgg tattaagaag tgaagtggga cccaggtgga ggtaaggaag agtctggtgg 60
   ggagttatct cagaagtgag gccagcacag gctgagtgca gccccagggt gagaaggtgc 120
   ccaccgagag gactctgcag tgaggcccta ggccacgtcc gaggggtgcc tccgatgggg 180
   gaagcccctc cctgggggtc accggagcca tgctgtcccg caagggcatc atccccgagg 240
   aatacgtgct gacacgcctg gcagaggacc ctgccgagcc caggtaccgt gcccgccagc 300
   ggagggcccg ctttgtgtcc aagaaaggca actgcaacgt ggcccacaag aacatccggg 360
   agcagggccg cttcctgcag gacgtgttca ccacgctggt ggacctcaag tggccacaca 420
   cattgctcat cttcaccatg tccttcctgt gcagctggct gctcttcgcc atggcctggt 480
   ggctcatcgc cttcgcccac ggtgacctgg cccccagcga gggcactgct gagccctgtg 540
   tcaccagcat ccactccttc tcgtctgcct tccttttctc cattgaggtc caagtgacta 600
   ttggctttgg ggggcgcatg gtgactgagg agtgcccact ggccatcctg agcctcatcg 660
   tgcagaacat cgtggggctc atgatcaacg ccatcatgct tggctgcatc ttcatgaaga 720
   ctgcccaagc ccaccgcagg gctgagaccc tcatcttcag caagcatgcg gtgatcgctc 780
   tgcgccacgg ccgcctctgc ttcatgctac gtgtgggtga cctccgcaag agcatgatca 840
   tcagcgccac catccacatg caggtggtac gcaagaccac cagccccgag ggcgaggtgg 900
   tgcccctcca ccaggtggac atccccatgg agaacggcgt gggtggcaac agcatcttcc 960
   tggtggcccc gctgatcatc taccatgtca ttgatgccaa cagcccactc tacgacctgg 1020
   cacccagcga cctgcaccac caccaggacc tcgagatcat cgtcatcctg gaaggcgtgg 1080
   tggaaaccac gggcatcacc acccaggccc gcacctccta cctggccgat gagatcctgt 1140
   ggggccagcg ctttgtgccc attgtagctg aggaggacgg acgttactct gtggactact 1200
   ccaagtttgg caacaccatc aaagtgccca caccactctg cacggcccgc cagcttgatg 1260
   aggaccacag cctactggaa gctctgaccc tcgcctcagc ccgcgggccc ctgcgcaagc 1320
   gcagcgtgcc catggccaag gccaagccca agttcagcat ctctccagat tccctgtcct 1380
   gagccatggt ctctcgggcc ccccacacgc gtgtgtacac acggaccatg tggtatgtag 1440
   cccagccagg gcctggtgtg aggctgggcc agcctcagct cagcctcccc ctgctgctca 1500
   tccagggtgt tacaaggcac ttgtcactat gctatttctg gcctcagcag gaacctgtac 1560
   tgggttattt ttgtccctgc tcctcccaac ccaatttagg actggctcac ccctctcccc 1620
   cgcccaaggc tgcag                                                  1635
<210> 7
   <211> 390
   <212> PRT
   <213> Homo sapiens
<400> 7
<210> 8
   <211> 1173
   <212> DNA
   <213> Homo sapiens
<400> 8
   atgctgtccc gcaagggcat catccccgag gaatacgtgc tgacacgcct ggcagaggac 60
   cctgccaagc ccaggtaccg tgcccgccag cggagggccc gctttgtgtc caagaaaggc 120
   aactgcaacg tggcccacaa gaacatccgg gagcagggcc gcttcctgca ggacgtgttc 180
   accacgctgg tggacctcaa gtggccacac acattgctca tcttcaccat gtccttcctg 240
   tgcagctggc tgctcttcgc catggcctgg tggctcatcg ccttcgccca cggtgacctg 300
   gcccccagcg agggcactgc tgagccctgt gtcaccagca tccactcctt ctcgtctgcc 360
   ttccttttct ccattgaggt ccaagtgact attggctttg gggggcgcat ggtgactgag 420
   gagtgcccac tggccatcct gatcctcatc gtgcagaaca tcgtggggct catgatcaac 480
   gccatcatgc ttggctgcat cttcatgaag actgcccaag cccaccgcag ggctgagacc 540
   ctcatcttca gcaagcatgc ggtgatcgcc ctgcgccacg gccgcctctg cttcatgcta 600
   cgtgtgggtg acctccgcaa gagcatgatc atcagcgcca ccatccacat gcaggtggta 660
   cgcaagacca ccagccccga gggcgaggtg gtgcccctcc accaggtgga catccccatg 720
   gagaacggcg tgggtggcaa cagcatcttc ctggtggccc cgctgatcat ctaccatgtc 780
   attgatgcca acagcccact ctacgacctg gcacccagcg acctgcacca ccaccaggac 840
   ctcgagatca tcgtcatcct ggaaggcgtg gtggaaacca cgggcatcac cacccaggcc 900
   cgcacctcct acctggccga tgagatcctg tggggccagc gctttgtgcc cattgtagct 960
   gaggaggacg gacgttactc tgtggactac tccaagtttg gcaacaccat caaagtgccc 1020
   acaccactct gcacggcccg ccagcttgat gaggaccaca gcctactgga agctctgacc 1080
   ctcgcctcag cccgcgggcc cctgcgcaag cgcagcgtgc ccatggccaa ggccaagccc 1140
   aagttcagca tctctccaga ttccctgtcc tga                               1173
<210> 9
   <211> 28
   <212> DNA
   <213> Artificial Sequence
<220>
   <223> Description of Artificial Sequence:primer
<400> 9
   rwardrmrma ggtggaggta aggaagag 28
<210> 10
   <211> 27
   <212> DNA
   <213> Artificial sequence
<220>
   <223> Description of Artificial sequence:primer
<400> 10
   rvrsrmrmgg tgaagatgag caatgtg 27
<210> 11
   <211> 28
   <212> DNA
   <213> Artificial Sequence
<220>
   <223> Description of Artificial Sequence:pnmer
<400> 11
   rwardrmrmg ggtggcaaca gcatcttc 28
<210> 12
   <211> 27
   <212> DNA
   <213> Artificial Sequence
<220>
   <223> Description of Artificial Sequence:primer
<400> 12
   rvrsrmrmtg gctcaggaca gggaatc 27
<210> 13
   <211> 3102
   <212> DNA
   <213> Homo sapiens
<300>
<400> 13
   stnsccaggt gctttttcag ggaccaagta gagctggggc ctgaccacag gcacttcttg 60
   gagccacaga cgcaaagcag cagccctcgg ggattgttct tccccagcca ccggcccaga 120
   gtgtggctgg tcaatcgtgg ggacccagga ctggctggac gcacagctct agggcccagt 180
   acctcccaca gcctctgcag ccttgggcgg gggagagggg tgagccagtc ctgaattggg 240
   ttgggaggag cagggacaaa aataacccag tacaggttcc tgctgaggcc agaaatagca 300
   tagtgacaag tgccttgtaa caccctggat gagcagcagg gggaggctga gctgaggctg 360
   gcccagcctc acaccaggcc ctggccgggc tacataccac atggtccgtg tgtacacacg 420
   cgtgtggggg gcccgagaga ccatggctca ggacagggaa tctggagaga tgctgaactt 480
   gggcttggcc ttggaccgag tcctgtccct tagmrvrscc atgggcacgc tgcgcttgcg 540
   caggggcccg cgggctgagg cgagggtcag agcttccagt aggctgtggt cctcatcaag 600
   ctggcgggcc gtgcagagtg gtgtgggcac tttgacggtg ttgccaaact tggagtagtc 660
   cacagagtaa cgtccgtcct cctcagctac aatgggcaca aagcgctggc cccacaggat 720
   ctcatcggcc aggtaggagg tgcgggcctg ggtggtgatg cccgtggttt ccaccacgcc 780
   ttccaggatg acgatgatct cgaggtcctg gtggtggtgc aggtcgctgg gtgccaggtc 840
   gtagagtggg ctgttggcat caatgacatg gtagatgatc agcggggcca ccaggaagat 900
   gctgttgcca cccacgccgt tctccatggg gatgtccact tctacgacaa cggtgggmrw 960
   ardcctggtg gaggggcacc acctcgccct cggggctggt ggtcttgcgt accacctgca 1020
   tgtggatggt ggcgctgatg atcatgctct tgcggaggtc acccacacgt agcatgaagc 1080
   agaggcggcc gtggcgcagg gcgatcaccg catgcttgct gaagatgagg gtctcagccc 1140
   tgcggtgggc ttgggcagtc ttcatgaaga tgcagccaag catgatggcg ttgatcatga 1200
   gccccacgat gttctgcacg atgaggatca ggatggccag tgggcactcc tcagtcacca 1260
   tgcgcccccc aaagccaata gtcacttgga cctcaatgga gaaaaggaag gcagacgaga 1320
   aggagtggat gctggtgaca cagggctcag cagtgccctc gctgggggcc aggtcaccgt 1380
   gggcgaaggc gatgagccac caggccatgg cgaagagcag ccagctgcac aggaaggaca 1440
   tggtgaagat gagccacttc tactccaatg tgtgtggcca cttgaggtcc accagcgtgg 1500
   tgaacacgtc ctgcaggaag cggccctgct cccgggttac amrvrsatgt tcttgtgggc 1560
   cacgttgcag ttgcctttct tggacacaaa gcgggccctc cgctggcggg cacggtacct 1620
   gggcttggca gggtcctctg ccaggcgtgt cagcacgtat tcctcgggga tgatgccctt 1680
   gcgggacagc atggctccgg tgacccccag ggaggggctt cccccatcgg aggcacccct 1740
   cggacgtggc ctagggcctc actgcagagt cctctcggtg ggcaccttct caccctgggg 1800
   ctgcactcag cctgtgctgg cctcacttct gagataactc cccaccagac tcttccttac 1860
   ctccacctgg gtcccacttc acttcttaat accagcgaga aggaatggag gtggamrwar 1920
   dctcaggccg ggcgcggtgg ctcacgcctg taatcccagt acgttgggag gctgaggagg 1980
   gcagatcact aggtcaggag ttcgagacca gcctgaccaa catggtgaaa ccccatctct 2040
   actaaaaata caaaagttag ccgggcatgg tggtgcgcac ctgtaatccc agctactcag 2100
   gaagctgagg caggagaatc gcttgaaccc gagaggcgga ggttgcagtg agccaagatc 2160
   acgccactgc actccagcct gggcgacaga gcgagactcc gtctcaaaaa caaaaaacaa 2220
   aacaaaaaaa acagcctcac ccttgagccc acctgcctgg cgcctaggga gcccccaacc 2280
   ctgccggctc tccacctggc cctgcgtcct cccccagctt cccccaccag ctgaccaccc 2340
   ctgggcctcc cgctaagagc ccttctcccc gcccagcctg ccttcccagc ccagctcaga 2400
   acttaacacc tccggatctc tcccactaac cggacctcac ccccacctcc gggtctcccg 2460
   cctcacctgt ctggccttcg ggctcccagg ctccagctca ggtcctggcc acccccgccc 2520
   cgtctctcct ccaggtgcgc cccctatgtc ctactccacc cctcccggag cgcccccgcc 2580
   gctttccgga tccccctcca gctctcgtcc tctctgtctc cttgggatcc tgcgttctct 2640
   ggagtcacca ccccacccgg ctcaaccgcg gctccccgga cctgcgcgcg acccggcggg 2700
   gctgactccg agtcgcggcc tcccggtccc tgccgcccgc ccgcgccgcg gctcccgctc 2760
   cgcgccgggt cccggccccg cccgccgccg ccgctgcacc tgctcctgct cctcacgcca 2820
   cggccgtcgc ctccccgccc cctcccgccg cccgggcgct cgcggctggc cgtcctcccg 2880
   tctgtctgtc cgtccgtggg tccgcggaga ccgacctggg ctaggcaggg gtctaggacg 2940
   gcgggagggg ggcgcacgag gagaggaacg gggccgggga ccgccagggg gagcccgacc 3000
   tgcaccggag cgcagggggc ggggccggag caagcccccg cccctcccgc gcccctcccc 3060
   gcccccgccc tccacctccc ctcccgggac tcacaacccg gc 3102